(19) Europäisches Patentamt
European Patent Office
Office européen des brevets

(11) EP 2 295 031 B1

(12) **EUROPÄISCHE PATENTSCHRIFT**

(45) Veröffentlichungstag und Bekanntmachung des Hinweises auf die Patenterteilung:
**10.01.2018 Patentblatt 2018/02**

(51) Int Cl.:
***A61K 8/49*** *(2006.01)* ***A61Q 19/06*** *(2006.01)*

(21) Anmeldenummer: **10170410.4**

(22) Anmeldetag: **22.07.2010**

(54) **Verwendung von Pterocarpanen als Anti-Cellulite-Wirkstoffe**

Use of pterocarpans as anti-cellulite agents

Utilisation de ptérocarpanes comme agents actifs anticellulite

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **05.08.2009 US 231473 P**

(43) Veröffentlichungstag der Anmeldung:
**16.03.2011 Patentblatt 2011/11**

(73) Patentinhaber: **Symrise AG**
**37603 Holzminden (DE)**

(72) Erfinder:
- **Meyer, Imke**
  **37619 Bodenwerder (DE)**
- **Koch, Oskar**
  **37079 Göttingen (DE)**
- **Hillebrand, Nadine**
  **32839 Steinheim (DE)**
- **Herrmann, Martina**
  **31789 Hameln (DE)**
- **Joppe, Holger**
  **37586 Dassel (DE)**

(74) Vertreter: **Eisenführ Speiser**
**Patentanwälte Rechtsanwälte PartGmbB**
**Postfach 10 60 78**
**28060 Bremen (DE)**

(56) Entgegenhaltungen:
**WO-A1-2007/128725**
**WO-A1-2009/060017**
**WO-A2-01/97771**
**CN-A- 101 244 020**
**JP-A- 2002 053 421**
**JP-A- 2008 222 622**
**US-A- 4 704 400**
**US-A- 6 030 620**
**US-A1- 2006 002 885**

- **LETICIA JIMÉNEZ-GONZÁLEZ ET AL: "Pterocarpans: interesting natural products with antifungal activity and other biological properties", PHYTOCHEMISTRY REVIEWS, KLUWER ACADEMIC PUBLISHERS, DO, Bd. 7, Nr. 1, 13. März 2007 (2007-03-13), Seiten 125-154, XP019556037, ISSN: 1572-980X, DOI: DOI:10.1007/S11101-007-9059-Z**
- **INGHAM AND P M DEWICK L: "The structure of desmocarpin, a pterocarpan phytoalexin from Desmodium gangeticum", ZEITSCHRIFT FUER NATURFORSCHUNG. SECTION C. BIOSCIENCES,, Bd. 39c, 1. Januar 1984 (1984-01-01), Seiten 531-534, XP009144026, ISSN: 0341-0382**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft primär die Verwendung bestimmter Pterocarpane der unten angegebenen Formel (I) als Anti-Cellulite Wirkstoffe und (kosmetische) Zubereitungen, die sich zur Prophylaxe und kosmetischen Behandlung von Cellulite beim Menschen eignen.

Cellulite ist auch unter den Synonyma Protrusio cutis und umgangssprachlich als Orangenhaut bekannt. Sie stellt ein kosmetisch-ästhetisches Problem dar, das mit Grübchenbildung und-Vertiefungen der Haut und Knötchenbildung des subkutanen Fettgewebes einhergeht. Cellulite kann an jeder Körperstelle des Menschen auftreten, am häufigsten sind jedoch die Außen- und Rückseite der Oberschenkel sowie das Gesäß betroffen. Auch Brüste, Unterbauch, Oberarme oder Nacken sind manchmal durch Cellulite geprägt. Klar zu trennen und zu unterscheiden von dem kosmetischen Phänomen der Cellulite ist die Cellulitis. Die Cellulitis ist eine bakterielle Infektion des Unterhautgewebes, welche in manchen Fällen eine schwere Erkrankung darstellen kann, und welche, anders als die Cellulite, therapeutisch behandelt werden muss.

Cellulite kann zwar regelmäßig an menschlichen Körperstellen mit übermäßiger Fettablagerung gefunden werden, doch stellt das Übergewicht keine Voraussetzung für deren Auftreten dar. Vermehrt haben auch schlanke Frauen ausgeprägte Cellulite-Erscheinungen. Zwischen der Schwere der Ausprägung der Cellulite und dem prozentualen Fettanteil im Gewebe besteht allerdings wohl eine Korrelation.

Die geschlechtsspezifische anatomische Struktur der Haut des Menschen hat einen großen Einfluss auf die Entstehung der Cellulite. So ist beim Mann Cellulite nur selten zu beobachten, dagegen sind 80% - 90% aller Frauen betroffen. Insbesondere der Aufbau der Dermis wirkt sich auf das Hautrelief aus. So werden beim Mann die Fettkammern beim Zusammenpressen der Haut durch sich überkreuzende Bindegewebssepten und den damit verbundenen klammerartigen Einschluss der Fettzellen zurückgehalten. Dagegen wölben sich bei der Frau die röhrenförmig voneinander getrennten Fettkammern, die durch radiär verlaufende Bindegewebssepten eingeschlossen sind, beim Zusammenpressen hoch.

Zudem beruht das sichtbare Bild der Cellulite auf einer Zunahme von Fettpolstern in der Subkutis sowie einer Minderung der Durchströmungsverhältnisse in den Blut- und Lymphbahnen. Die Ursache ist somit eine zum Teil anlagebedingte Schwächung des Bindegewebes mit gleichzeitigem Auftreten von vergrößerten Fettzellkammern, wobei neben genetischen Faktoren Stress, sportliche Aktivität, Rauchen, Schwangerschaften sowie weibliche Hormone (Östrogen und Progesteron) eine Rolle spielen.

Die herkömmlichen Behandlungsmethoden der Cellulite versuchen die Durchblutung der betroffenen Hautpartien zu fördern und die Bindegewebsstruktur positiv zu beeinflussen, beispielsweise durch Massage, Lymphdrainage, Diät, Sport, Magnetfelder oder auch Liposuction (Fettabsaugung).

In der Literatur wird die Verwendung von Phosphodiesterase-Inhibitoren zur Stimulation der Lipolyse in den Fettzellen beschrieben (Cellular Signalling 2006, 18, 401-8). Laut Biochem. Pharmacol. 1992, 44, 1307-1315 wird die Lipolyse nicht grundsätzlich durch Phosphodiesterase-Inhibitoren stimuliert, da die Lipolyse durch verschiedene Faktoren beeinflusst wird.

Die Regulierung des Fettstoffwechsels im Fettgewebe des Menschen zur Reduzierung der eingelagerten Lipidmenge kann prinzipiell auf drei Wegen erfolgen:

Weg (i): Die Differenzierung der als Preadipozyten bezeichneten Vorläuferzellen der Fettzellen zu den eigentlichen Fettzellen, Adipozyten genannt, die Triglyceride einlagern können, kann gehemmt werden. Vereinfacht ausgedrückt verhindert eine Hemmung des Wegs (i) den Aufbau der Cellulite dadurch, dass die Anzahl der Fettzellen nicht zunimmt.

Weg (ii): Die Einlagerung von Triglyceriden in den Adipozyten - auch als Lipogenese bezeichnet - kann gehindert bzw. inhibiert werden. Vereinfacht ausgedrückt verhindert eine Hemmung des Wegs (ii) die Einlagerung weiterer Triglyceride (Fette) in der Zelle, vorhandene Fettzellen lagern kein neues Fett ein. Durch den natürlichen Fettstoffwechsel nimmt bei Hemmung des Wegs (ii) der Fettgehalt in der Zelle ab.

Weg (iii): Eine vermehrte / verstärkte Hydrolyse bereits in den Adipozyten eingelagerter Lipide - auch als Lipolyse bezeichnet - ist durch eine gezielte Stimulation möglich. Vereinfacht ausgedrückt verstärkt eine Stimulierung des Wegs (iii) den Abbau der bereits in der Zelle vorhandenen Fette, eine hemmende / inhibierende, d.h. antagonistische, Wirkung bezüglich des Wegs (iii) hingegen be- bzw. verhindert den Fettabbau.

Die Differenzierung von Zellen ist die Veränderung der Regulierung der Genaktivität einer Zelle, so dass über Transkription und Protein-Biosynthese verschiedene Protein-Bestände in den Zellen realisiert werden und sich die Zellen nach Aussehen und Funktion unterscheiden. So exprimieren Adipozyten erst nach der Differenzierung Enzyme, die für die Einlagerung von Fetten notwendig sind. In ihren Vorläuferzellen undifferenzierten Preadipozyten werden diese Enzyme nicht oder nur in sehr geringem Umfang exprimiert.

Kosmetische Zubereitungen, welche die Prophylaxe und Behandlung von Cellulite zum Ziel haben, sind in der Literatur bereits vorgeschlagen worden.

In EP 1 234 572 wird eine kosmetische Zubereitung aus mindestens einem Isoflavon-Aglykon aus der Gruppe

Genistein, Daidzein, Glycitein, Formononetin, Tectorigenin, Irigenin, Biochanin A, O-Desmethylangolensin, Equol, Orobol, Santal, Pratensin und Apiosylpuerarin, insbesondere Genistein und/oder Daidzein, zur Behandlung von Cellulite beschrieben. Das Isoflavon-Aglykon wird dabei mit einem Algenextrakt kombiniert. Genistein wird dort als ein Wirkstoff beschrieben, der die Vermehrung von Vorläufer-Fettzellen und zudem das Enzym Phosphodiesterase hemmt.

In DE 10 2004 032 837 wird eine kosmetische Zubereitung aus bestimmten Biochinonen und Isoflavonoiden, bevorzugt Genistein, zur Prophylaxe von Cellulite beschrieben. Es wird behauptet, dass die Wirkung dieser Zubereitung über eine Verbesserung des Zellstoffwechsels erfolgt. Es ist dabei nicht ersichtlich, welcher Mechanismus des Zellstoffwechsels verbessert wird. Es ist dort auch nicht zu entnehmen, ob das Fettgewebe durch die kosmetische Zubereitung beeinflusst wird.

Ebenfalls Zubereitungen bestimmte Isoflavone enthaltend werden in DE 100 09 423 beschrieben, wobei die Isoflavone als Stoffe mit antiöstrogener Wirkung beschrieben und aufgrund dieser Wirkung eingesetzt werden. Als Isoflavone bevorzugt sind dabei Daidzein, Genistein, Glycitein, Formononetin und andere.

WO 01/97771 A2 offenbart die Bereitstellung von Extrakten der Pflanze *Pistia stratiotes* für die Entwicklung neuer Haut- und Haarpflegemittel. Die eingesetzten Pflanzenextrakte, welche keine Verbindungen der Formel (I) enthalten, weisen schlankmachende und anti-Cellulitis-Eigenschaften auf und können zudem zur Behandlung von Alterserscheinungen der Haut eingesetzt werden. Die Offenlegungsschrift beschreibt zudem im Beispielteil, dass die offenbarten Extrakte die Lipolyse-Aktivitat in humanen Adipozyten anregen.

US 6,030,620 beschreibt die Verwendung von aus Kichererbsen extrahierten Phytoöstrogenen zur Erhöhung der Östrogenaktivitat in der Haut sowie zur Vermehrung von Fibroblasten in der Haut sowie zur Vermehrung von Hautzellen in der Epidermis.

Nachteilig am Stand der Technik ist der Umstand, dass die bekannten Zubereitungen nur eine begrenzte Wirksamkeit bei der Prophylaxe und Behandlung von Cellulite aufweisen. So zeigte Genistein in unseren eigenen umfangreichen Untersuchungen einen stark von der Konzentration abhängigen Effekt auf die Differenzierung der Fettzellen.

Je nach Einsatzkonzentration von Genistein wird die Differenzierung der Fettzellen inhibiert oder stimuliert. Die Stimulation der Differenzierung von Vorläuferzellen ist bei der Behandlung von Cellulite nicht erwünscht, da das Bild der Cellulite dadurch verstärkt wird. Bei der Herstellung und in vivo-Anwendung von Zubereitungen Genistein enthaltend muss somit sehr sorgsam die Konzentration ausgewählt werden und die Hautpenetration durch die verwendete Formulierung beachtet werden, um nicht den antagonistischen Effekt der Differenzierungs-Stimulation zu erzielen.

So zeigt bei eigenen Untersuchungen ein Teil der in der oben genannten Patentliteratur beschriebenen Isoflavone antagonistische Effekte hinsichtlich der Lipolyse von Fettzellen. Als Beispiel seien die Isoflavone Santal und Formononetin genannt, die gemäß EP 1 234 572 zur Behandlung von Cellulite (dort als "Zellulitis" bezeichnet) einzusetzen sind, in unseren eigenen Untersuchungen jedoch eine Inhibition der Lipolyse zeigten.

Diese Isoflavone stimulieren nicht die Lipolyse (Weg iii), sondern inhibieren den Abbau der in der Fettzelle bereits eingelagerten Triglyceride, sie zeigen also hinsichtlich der Lipolyse eine antagonistische Wirkung. Somit kann nicht aufgrund einer Inhibition der Phosphodiesterase auf die Stimulation der Lipolyse geschlossen werden.

Es war daher die Aufgabe der vorliegenden Erfindung Wirkstoffe und entsprechende Zubereitungen anzugeben, welche eine, vorzugsweise verbesserte, Wirksamkeit zur Prophylaxe und Behandlung von Cellulite aufweisen.

Überraschenderweise hat sich gezeigt, dass diese Aufgabe gelöst werden kann durch die Anti-Cellulite-Wirkstoffe der Formel (I) (Pterocarpane) sowie entsprechende Zubereitungen, insbesondere kosmetische, dermatologische oder pharmazeutische Zubereitungen, enthaltend eine wirksame Menge einer oder mehrerer Verbindungen der Formel (I)

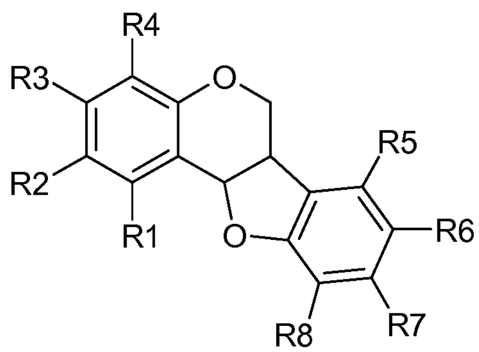

(I)

wobei

R1 bis R8, unabhängig voneinander, Wasserstoff, Hydroxy oder $C_1$-$C_4$-Alkoxy bedeuten können und jeweils zwei unmittelbar benachbarte Reste von R1 bis R8 über eine Methylendioxygruppe -O-$CH_2$-O- verbunden sein können. Soweit also ein Rest R1 bis R8 nicht zusammen mit einem der weiteren Reste R1 bis R8 eine Methylendioxygruppe bildet, ist dieser Rest Wasserstoff, Hydroxy, Methoxy, Ethoxy, n-Propoxy, iso-Propoxy, n-Butoxy, iso-Butoxy, sec-Butoxy oder tert-Butoxy.

Die Verbindungen der Formel (I) zeigen eine ausgeprägte Wirkung bei der Behandlung von Cellulite, erkennbar mittels echographischer Bestimmung der Subcutis-Schichtdicke, insbesondere zur Vorbeugung der verstärkten Bildung von Fettdepots in der Haut und/oder Cellulite, indem der Lipidgehalt im humanen Unterhaut-Fettgewebe reduziert wird. Die Erfindung betrifft daher die kosmetische Verwendung einer Zubereitung enthaltend eine entsprechende wirksame Menge an einer oder mehreren Verbindungen der Formel (I), insbesondere zur topischen Behandlung und Vorbeugung der verstärkten Bildung von Fettdepots in der Haut und/oder Cellulite.

Bevorzugt bedeuten R1 bis R8, unabhängig voneinander, Wasserstoff, Hydroxy, Methoxy oder Ethoxy, soweit nicht jeweils zwei benachbarte Reste von R1 bis R8 gemeinsam eine Methylendioxygruppe -O-$CH_2$-O- bilden, und wobei weiter bevorzugt R3 nicht Wasserstoff ist.

Alternativ oder zusätzlich haben vorzugsweise höchstens drei, bevorzugt höchsten zwei, der Reste R1 bis R8 gleichzeitig die Bedeutung Hydroxy.

Weiter erfindungsgemäß bevorzugt ist die Verwendung einer Zubereitung wie vorangehend beschrieben, wobei eine Verbindung der Formel (I) eine Verbindungen der Formel (IA) ist

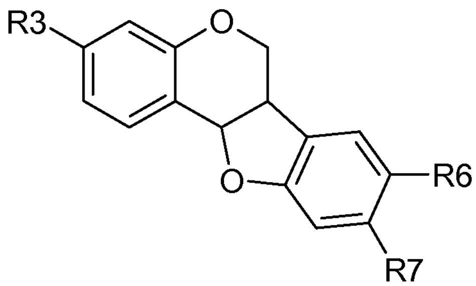

(IA)

wobei der Rest R3 Wasserstoff, Hydroxy oder Methoxy bedeutet und die Reste R6 und R7 gemeinsam eine Methylendioxygruppe bilden oder unabhängig voneinander Wasserstoff, Hydroxy oder Methoxy bedeuten, und wobei weiter bevorzugt R3 Hydroxy oder Methoxy ist.

Insbesondere bevorzugt ist die Verwendung einer Zubereitung wie vorangehend beschrieben, wobei eine, mehrere oder alle Verbindungen der Formel (I) ausgewählt sind aus der Gruppe bestehend aus Medicarpin, Maackiain, Pterocarpin, Homopterocarpin und 3-Methoxypterocarpan (entsprechend Verbindung der Formel (B)). Am meisten bevorzugt für die Zwecke der Erfindung ist die Verbindung der Formel (B).

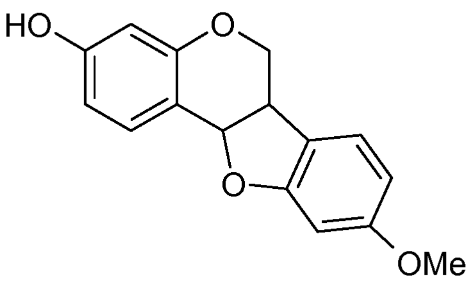

Medicarpin

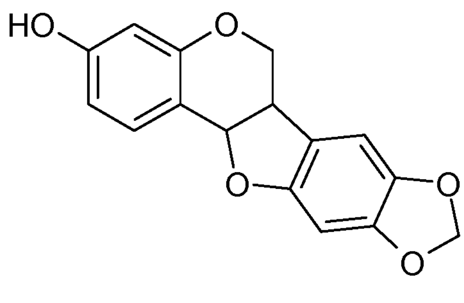

Maackiain

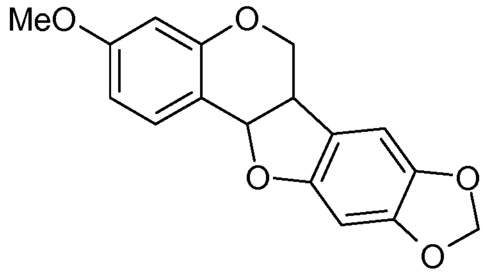

Pterocarpin

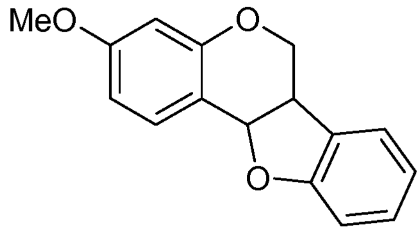

(B)

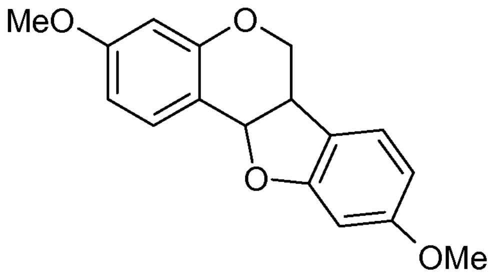

Homopterocarpin

Die Verbindungen der Formel (I) sind strukturell den Pterocarpanen zuzuordnen. Es handelt sich zum Teil um soganannte Phytoalexine, natürliche Verbindungen die von Pflanzen in Folge einer Infektion gebildet werden und zur Abwehr dienen. Zu den Pflanzen, die Pterocarpane bilden, zählen beispielsweise Arten der Gattung Sophora. Die natürlich vorkommenden Pterocarpane Maackiain und Medicarpin sind als Komponenten von häufig in der traditionellen Medizin verwendeten Pflanzen wie Sophora flavescens, Sophora subprostata und Sophora japonica bereits umfangreich untersucht worden. So sind unter anderem antipyretische, fungizide, antibakterielle und Antitumor-Wirkungen in der Literatur beschrieben, ebenso wie Wirkungen gegen Hefen und Schlangengift (siehe Eintrag im RÖMPP-Lexikon und die in der US 5,721,371 zitierten Literaturstellen).

JP 2002-053421 beschreibt bestimmte Pterocarpane, insbesondere aufgereinigtes Maackiain aus Wurzeln von Sophora subprostata, für die Hemmung des Dendritenwachstums von Melanozyten, d.h. zur Beeinflussung der Hautpigmentierung.

Eine Inhibition der Leukotrien-Biosynthese ist aus US 4,704,400 für Medicarpin und Derivate isoliert aus Dalbergia odorifera bekannt.

Verschiedene Pterocarpane sowie Pterocarpane enthaltende Extrakte werden in WO 2007/128725 als Antagonisten des Arylhydrocarbon-Rezeptors beschrieben, die dort auch in kosmetischen Zuereitungen eingesetzt werden.

Einige Pterocarpane sind in Leguminosen vorkommende Naturstoffe mit dem *cis*-6a,11a-Dihydro-6*H*-benzofuro[3,2-*c*][1]benzopyran-Ringsystem (Pterocarpan). Wichtige Vertreter sind beispielsweise Maackiain und Pterocarpin. In der Natur kommen sowohl beide Enantiomere als auch die Racematformen vor. Der Gehalt an Pterocarpanen in Pflanzen ist zumeist gering. Eine Isolierung zur Nutzung der Pterocarpane für kosmetische Zubereitungen ist technisch zwar möglich, aufgrund der geringen Ausbeute jedoch wirtschaftlich nicht sinnvoll.

Ein Syntheseweg, der sich zur Herstellung der Pterocarpane der Formel (I) eignet, ist in US 5,721,371 beschrieben worden.

Eine Enantiomeren-Trennung mittels HPLC einiger Pterocarpane, unter anderem von Verbindung (B), ist in J. Liquid Chromatography & Related Technologies 2005, 28, 823-834 beschrieben.

Ebenfalls lassen sich die Verbindungen der Formel (I), insbesondere die der Formel (IA) und (B), gemäß oder in Anlehnung an Tetrahedron 1999, 55, 11773-11786 herstellen.

Ferner können die Verbindungen der Formel (I) gemäß oder in Anlehnung an Synlett 1994, 1001-1002, J. Chem. Soc., Perkin Trans 1, 1998, 2533-2536 und J. Nat. Prod. 2008, 71, 275-277 hergestellt werden.

Die erfindungsgemäß einzusetzenden Verbindungen der Formel (I) können auch in Form von Extrakten aus Pflanzen eingesetzt werden. Die erfindungsgemäß einzusetzenden Extrakte werden vorzugsweise aus dem Holz oder Kernholz der Pflanzen des Genus Bobgunnia, bevorzugt aus der Pflanze Bobgunnia madagascariensis, gewonnen, die zur Familie Fabaceae, Unterfamilie der Schmetterlingsblütler (Faboideae), Tribus Swartzieae, zählt. Diese Pflanze ist

auch unter der Bezeichnung Swartzia madagascariensis bekannt, obwohl bereits 1997 der Genus Bobgunnia vom Genus Swartzia abgespalten wurde (Kirkbride et al. Brittonia 1997, Seiten 1-23). Eine gebräuchliche Bezeichnung ist zudem das Synonym Pao Rosa. Bei der Pflanze handelt es sich um einen regelmäßig 4 - 9 m (bis zu 20 m) hohen Baum oder Strauch mit charakteristischen weißen Blüten, buschig oder mit kurzem Stamm bis 40 cm Durchmesser. Das Kernholz ist purpur-schwarz, hart, dauerhaft und termitenfest. Die Pflanze ist von der Sahelzone bis zum nordöstlichen Namibia verbreitet. (Hans Dieter Neuwinger: Afrikanische Arzneipflanzen und Jagdgifte, Chemie, Pharmakologie, Toxikologie, Wiss. Verl.-Ges. 1998, Seiten 316-321).

In der traditionellen Medizin sind je nach Land und Stamm verschiedene Anwendungen zu finden. So haben die Wurzeln den Ruf großer Wirksamkeit bei Syphilis und Lepra; gekochte Wurzeln werden bei Gastritis eingesetzt; ein Rindenaufguss wird bei Gelbsucht verordnet; der Wurzel-Aufguss wird bei Ödemen getrunken; das Wurzelpulver wird bei Diarrhoe gegessen. (Hans Dieter Neuwinger: Afrikanische Arzneipflanzen und Jagdgifte, Chemie, Pharmakologie, Toxikologie, Wiss. Verl.-Ges. 1998, Seiten 316-321)

Die Verwendung eines Extraktes aus den Schoten und Samen als Molluskizid ist beschrieben und auf die enthaltenen Saponine zurückzuführen (Suter et al., Acta Tropica 1986, Seiten 69-83; Borel et al., Helvetica Chimica Acta 1987, Seiten 570-576).

Georges et al. (Bioresource Technology 2008, Seiten 2037-2045) beschreiben die Verwendung eines Extraktes aus Blättern von Swartzia madagascariensis als Insektenschutzmittel.

Eine Aktivität durch den Extrakt der Wurzelrinde von Bobgunnia madagascariensis wurde gegen die intrazelluläre Form von Leishmania major in Journal of Ethnopharmacology 2007, Seiten 99-104 beschrieben.

In US 5,929,124 wird die Verwendung eines Extraktes der Wurzelrinde aufgrund antimikrobieller Aktivität gegen Candida, Aspergillus, Staphylococccus etc. beschrieben und die Aktivität auf enthaltene Diterpene zurückgeführt. Eine Verwendung für Haut, Haare und Nägel bei mikrobiellen Infektionen wird beschrieben, wobei der aus der Wurzelrinde hergestellte Extrakt bzw. die daraus isolierten Diterpene auch topisch appliziert werden können.

Eine antimikrobielle Aktivität eines Ethanol-Extraktes der Wurzeln von Bobgunnia madagascariensis wird durch Kone et al. (Journal of Ethnopharmacology 2004, Seiten 43-49) beschrieben.

Ebenfalls antimikrobielle Aktivität ist für Extrakt-Mischungen, die einen Methanol-Extrakt der Rinde von Swartzia madagascariensis enthielten, durch Magassouba et al. (Journal of Ethnopharmacology 2007, Seiten 44-53) festgestellt worden.

Das Kernholz von Pao Rosa wurde von Harper et al. (J. Chem. Soc. 1969, Seiten 1109-1116) hinsichtlich enthaltener Inhaltsstoffe untersucht, wobei eine Identifikation lediglich für den Ether-Extrakt sowie für den Petroleum Ether-Extrakt vorgenommen wurde. Ein Ethanol-Extrakt des zuvor mit Petroleum und Ether extrahierten Kernholzes wurde hergestellt, die enthaltenen Komponenten aber nicht weiter untersucht. In den untersuchten Extrakten des Kernholzes von Pao Rosa sind verschiedene Pterocarpane, darunter Homopterocarpin, Pterocarpin, 3,9-Dimethoxy-Pterocarpen sowie 3,4,9-Trimethoxy-Pterocarpan, nachgewiesen worden.

In WO 2007/128725 A1 werden Pterocarpane sowie Extrakte Pterocarpane enthaltend als Aryl hydrocarbon Receptor (AhR) Antagonisten und ihre Verwendung in kosmetischen Formulierungen beschrieben. Extrakte aus Holz - bevorzugt Kernholz - der Gattung Swartzia sowie als Beispiel Swartzia madagascariensis werden ebenfalls genannt. Die Extraktion erfolgt dabei mit einem Extraktionsmittel enthaltend mindestens eines der folgenden Lösungsmittel: Wasser, Ethylacetat, ein Alkohol und/oder ein Keton ausgewählt aus der Gruppe Methanol, Ethanol, n-Propanol, Isopropanol und Aceton. Dort wird eine weitere Behandlung des Extraktes nicht vorgenommen, sondern dieser direkt als Rohextrakt eingesetzt.

Es hat sich in unseren eigenen Untersuchungen jedoch gezeigt, dass der gemäß WO 2007/128725 A1 hergestellte Rohextrakt aus Pao Rosa-Kernholz phototoxische Eigenschaften aufweist, wodurch dieser für den Einsatz in kosmetischen Zubereitungen wenig bis nicht geeignet ist. In eigenen Untersuchungen konnte weiter gezeigt werden, dass Rohextrakte aus Pao Rosa nach einer Behandlung mit Aktivkohle im Anschluss an die Extraktion kein (nennenswerte) phototoxisches Potential mehr aufweisen. In dem erfindungsgemäßen Pao Rosa Kernholz Extrakt sind neben Pterocarpanen auch Isoflavone wie beispielsweise Afrormosin nachgewiesen worden. Das Isoflavon Afrormosin und andere methoxylierte Isoflavone (Odoratin, Cladastrin und andere) weisen ein merkliches phototoxisches Potential auf. In dem hierin beschriebenen Pao Rosa Extrakt ist durch die Behandlung mit Aktivkohle der Gehalt von Afrormosin und anderen Isoflavonen reduziert, wodurch auch das phototoxische Potential reduziert wird.

Das phototoxische Potential wird als Photo-Irritations-Faktor (PIF) ausgedrückt. Der PIF eines Pflanzenextraktes enthaltend eine oder mehrere Verbindungen der Formel (I), vorzugweise Extrakte aus (Kern)Holz der Pflanzen der Gattung Bobgunnia, bevorzugt Pao Rosa, wird auf einen Wert von kleiner als 5 eingestellt, vorzugsweise auf einen Wert kleiner als 2, gemäß OECD Guideline 432 in der Fassung vom 13. April 2004. Dabei gilt, dass bei einem PIF von kleiner 2 kein phototoxisches Potential besteht, bei einem PIF größer 5 phototoxisches Potential besteht. Bei einem PIF zwischen 2 und 5 liegt möglicherweise ein phototoxisches Potential vor. Die erfindungsgemäßen Extrakte, bevorzugt aus Pflanzen der Gattung Bobgunnia, insbesondere Pao Rosa-Kernholz - Extrakte, weisen nach Behandlung mit Aktivkohle einen PIF von kleiner 2 auf, d.h. dass diese gemäß OECD Guideline 432 kein phototoxisches Potential mehr aufweisen.

Ein Extrakt wird aus Holz bzw. Kernholz der Pflanzen der Gattung Bobgunnia, bevorzugt aus Pao Rosa-Kernholz, enthaltend Verbindungen der Formel (I), insbesondere die Verbindungen Maackiain und Medicarpin, hergestellt. Als Trockenextrakt ist ein Extrakt hergestellt oder herstellbar nach dem nachfolgenden Verfahren nach vollständiger Entfernung des Extraktionsmittels anzusehen. Das Verfahren zum Herstellen eines Extraktes aus Pao Rosa-Kernholz umfasst folgende Schritte:

(1) Bereitstellen von Holz, vorzugsweise von Holzraspat, von Pflanzen des Genus Bobgunnia,
(2) Versetzen des in Schritt (1) bereitgestellten Holzes mit einem Extraktionsmittel ausgewählt aus

A) der Gruppe bestehend aus Methanol, Ethanol, n-Propanol, Isopropanol und Mischungen zweier oder mehrerer dieser Extraktionsmittel, oder
B) der Gruppe bestehend aus Mischungen von Wasser und einer oder mehrerer der Substanzen Methanol, Ethanol, n-Propanol und Isopropanol,

(3) bis zu 24h Extrahieren des in Schritt (2) mit dem Extraktionsmittel versetzten Holz zum Gewinnen eines Rohextraktes, und
(4) Behandeln des in Schritt (3) gewonnenen Rohextraktes mit Aktivkohle zum Gewinnen des Extraktes mit einem Photo-Irritationsfaktor gemäß OECD Guideline 432 in der am 13. April 2004 angenommenen Fassung von weniger als 5, bevorzugt von weniger als 2.

Dabei werden als Holzraspat vorzugsweise gewählt: Holzspäne, beispielsweise Hobelspäne, und/oder Holzmehl, beispielsweise Sägemehl, und/oder auf andere Weise geraspeltes Holz.

Das Verhältnis der Masse an Extraktionsmittel zu Holzraspatmasse wird bevorzugt so eingestellt, dass zumindest die 2-fache Masse Extraktionsmittel bezogen auf die Holzraspatmasse und vorzugsweise nicht mehr als die 20-fache Masse Extraktionsmittel bezogen auf die Holzraspatmasse erreicht wird. Besonders bevorzugt wird die 2- bis 10-fache Masse Extraktionsmittel bezogen auf die Holzraspatmasse zur Extraktion eingesetzt. Die besten Ergebnisse konnten mit einer 5-fachen Masse eines ethanolhaltigen Lösungsmittels (wiederum bezogen auf die Holzraspatmasse) erreicht werden.

Die Extraktionszeit zum Durchführen von Schritt (3) beträgt höchstens 24 Stunden, kann jedoch auch kürzer sein. Es ist bevorzugt, das Holzraspat in Schritt b) zumindest 1 h lang, insbesondere zumindest 2 h lang zu extrahieren. Die zum Herstellen eines Extrakts für die Verwendung in kosmetischen Zubereitungen benötigte Extraktionszeit beträgt vorzugsweise höchstens 24 h und besonders bevorzugt höchstens 4 h. Die Wahl der benötigten Extraktionszeit erfolgt in Abhängigkeit von der Qualität des zu extrahierenden Holzraspats und von den übrigen Extraktionsbedingungen, insbesondere der Extraktionstemperatur.

Zusätzlich ist es besonders bevorzugt, die Extraktion in Schritt (3) unter Rückfluss des Extraktionsmittels durchzuführen. Die Extraktionstemperatur wird in Abhängigkeit vom verwendeten Extraktionsmittel eingestellt. Bei Verwendung eines ethanolhaltigen Lösungsmittels ist eine Extraktionstemperatur von 80 - 100 °C bevorzugt.

Um die Phototoxizität des Extraktes zu reduzieren, wird nach der Extraktion in Schritt (4) der Rohextrakt mit Aktivkohle versetzt. Das Gewichtsverhältnis von Aktivkohle zu in Schritt (2) eingesetztem Holzraspat ist dabei vorzugsweise so gewählt, dass dieses von 1 : 1 bis 1 : 50, bevorzugt von 1 : 2 bis 1 : 20, und besonders bevorzugt von 1 : 5 bis 1 : 10 liegt.

Durch die Behandlung des Rohextraktes mit Aktivkohle in Schritt (4) nimmt der Gehalt der Verbindungen der Formel (I), insbesondere der Verbindungen Maackiain und Medicarpin, zu. Dagegen wird der Gehalt des Isoflavons Afrormosin, das für ein hohes phototoxisches Potential bekannt ist, reduziert.

Der Extrakt sollte für die Verwendung in kosmetischen Zubereitungen frei von Extraktionsmittel sein. Das Extraktionsmittel sollte durch ein geeignetes Verfahren (z.B. Destillation, Trocknung, Gefriertrocknung) möglichst vollständig entfernt werden.

Die erfindungsgemäß zu verwendenden Zubereitungen enthaltend eine oder mehrere Verbindungen der Formel (I) beeinflussen die Cellulite im Hinblick auf die eingelagerte Lipidmenge, indem der Lipidgehalt im humanen Unterhaut-Fettgewebe reduziert wird.

Außerdem wirken die erfindungsgemäß zu verwendenden Zubereitungen positiv auf Hautreizungen, die mit einer Cellulite einhergehen können.

So wird Cellulite durch die Verwendung einer Zubereitung enthaltend eine oder mehrere Verbindungen der Formel (I) durch die Beeinflussung der oben beschriebenen Wege (i) und (ii) vorgebeugt, behandelt oder verringert. Die Lipolyse (Weg (iii)) wird durch die Verbindungen der Formel (I) nicht beeinflusst. Im Unterschied zu den im oben zitierten Stand der Technik genannten Isoflavonen beeinflussen die Verbindungen den Weg (iii) nicht antagonistisch.

Es versteht sich, dass anstelle von Zubereitungen enthaltend Reinstoffe der Formel(I) auch Zubereitungen enthaltend einen Extrakt aus Pao Rosa Kernholz erfindungsgemäß verwendbar sind. Auch solche Zubereitungen beeinflussen die Cellulite durch Beeinflussung der Wege (i) und (ii). Der Extrakt aus Pao Rosa Kernholz zeichnet sich dabei aus durch einen hohen Gesamt-Gehalt an Pterocarpanen, insbesondere Maackiain und Medicarpin, von 5 Gew.-% bis 25 Gew.-%, bezogen auf den getrockneten Extrakt.

Vorzugsweise enthält eine erfindungsgemäß zu verwendende kosmetische, vorzugsweise topische, Zubereitung eine oder mehrere Verbindungen der Formel (I) ohne Berücksichtigung eventueller Gegenionen in einer Gesamtmenge von 0,001 - 1,0 Gew.-%, bevorzugt von 0,005 - 0,5 Gew.-%, und besonders bevorzugt von 0,01 - 0,15 Gew.-%, und am meisten bevorzugt von 0,02 - 0,10 Gew.-%, jeweils bezogen auf das Gesamtgewicht der Zubereitung.

Alternativ bevorzugt ist eine erfindungsgemäß zu verwendende Zubereitung enthaltend einen erfindungsgemäßen Pao Rosa-Kernholz Trockenextrakt im Bereich von 0,001 - 5 Gew.-%, bevorzugt in einer Konzentration von 0,01 - 0,50 Gew.-%, weiter bevorzugt in einer Konzentration von 0,02 - 0,20 Gew.-%, jeweils bezogen auf das Gesamtgewicht der Zubereitung.

Die Verbindungen der Formel (I), ggf. in Form eines Extraktes, lassen sich in diesen Konzentrationen gut in gängige kosmetische oder dermatologische Formulierungen wie Pumpsprays, Aerosolsprays, Cremes, Salben, Tinkturen, Lotionen und dergleichen einarbeiten. Hierbei ist es auch möglich und in manchen Fällen vorteilhaft, die Verbindungen der Formel (I) mit weiteren Wirkstoffen zu kombinieren.

Die Erfindung betrifft folglich die Verwendung (verbesserter) kosmetischer Zubereitungen enthaltend:

(a) eine oder mehrere Verbindungen der Formel (I), gegebenenfalls in Form eines Pflanzenextraktes, vorzugsweise der Gattung Bobgunnia,
und

(b) eine oder mehrere Lipolyse-Stimulantien.

Lipolyse-Stimulantien sind Wirkstoffe, die die Lipolyse (Weg (iii)) stimulieren, und sind vorzugsweise gewählt aus der Gruppe (b-i) der Inhibitoren der Phosphodiesterase, und/oder
der Gruppe (b-ii) der Agonisten beta-adrenerger-Rezeptoren.

Sinnvollerweise liegt das Lipolyse-Stimulans in einer zum Stimulieren der Lipolyse ausreichenden Menge vor.

Eine vorteilhafte erfindungsgemäß zu verwendende Zubereitung enthält zusätzlich Anti-Cellulite-Wirkstoffen der Gruppe der Xanthine, vorzugsweise gewählt aus der Gruppe der gegebenenfalls substituierten 3,7- oder 3,9-Dihydro-1*H*-purin-2,6-dione der Formel (Xa):

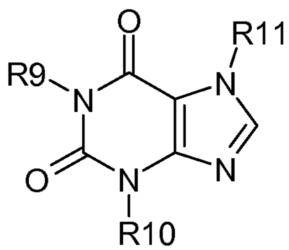

(Xa)

wobei R9, R10 und R11 unabhängig voneinander Wasserstoff oder Methyl bedeuten.

Die Xanthine, insbesondere die der Formel (Xa), können vorzugsweise als Reinstoffe oder auch in Form von Pflanzenextrakten eingesetzt werden.

Bevorzugt sind die Methylxanthine Coffein (R9 = R10 = R11 = $CH_3$), Theobromin (R9 = H, R10 = R11 = $CH_3$) und Theophyllin (R9 = R10 = $CH_3$, R11 = H), das am meisten im Sinne der vorliegenden Erfindung bevorzugte Xanthin ist Coffein. Ebenfalls bevorzugt ist das Theophyllin-Derivat Aminophyllin.

Besonders bevorzugt enthält eine kosmetische, vorzugsweise topische, erfindungsgemäß zu verwendende Zubereitung eine oder mehrere Verbindungen der Formel (Xa), dabei wiederum bevorzugt Coffein, vorzugsweise in einer Gesamtmenge von 0,005 - 10 Gew.-%, bevorzugt von 0,05 - 5 Gew.-%, besonders bevorzugt von 0,5 - 2,5 Gew.-%, jeweils bezogen auf das Gesamtgewicht der Zubereitung, wobei Gegenionen der Verbindungen der Formel (Xa) nicht mitgerechnet werden.

Bevorzugte Gewichtsverhältnisse der Gesamtmenge der Verbindung der Formel (I) zu der Gesamtmenge der Xanthine der Formel (Xa), dabei bevorzugt Coffein, in den erfindungsgemäßen Zubereitungen liegen bevorzugt von 1 : 1 bis 1 : 500, weiter bevorzugt von 1 : 5 bis 1 : 125, ebenfalls ohne Berücksichtigung eventueller Gegenionen.

Ebenfalls bevorzugt zu verwendende Zubereitungen enthalten Kombinationen der Verbindung der Formel (I)

mit einem Agonisten beta-adrenerger-Rezeptoren der Adipozyten. Bevorzugte Agonisten beta-adrenerger-Rezeptoren sind β-Phenylethylamine der Formel (PhEA):

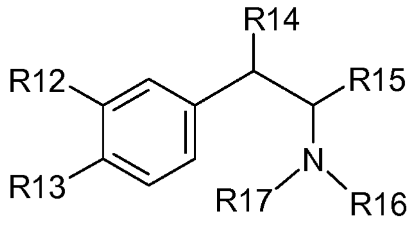

(PhEA)

wobei

R12 und R13, unabhängig voneinander, Wasserstoff, Hydroxy oder Methoxy,

R14 Wasserstoff, Hydroxy oder Methyl,

R15 Wasserstoff oder Methyl,

R16 und R17, unabhängig voneinander, Wasserstoff oder $C_1$-$C_4$-Alkyl,

bedeuten.

Die β-Phenylethylamine der Formel (PhEA) können vorzugsweise als Reinstoffe, in Form ihrer jeweiligen Hydrochloride oder in Form von Pflanzenextrakten eingesetzt werden.

Bevorzugte Agonisten beta-adrenerger-Rezeptoren sind Adrenalin, Noradrenalin, Metanephrin, Macromerin, Normacromerin, Hordenin, N-Methyltyramin, Dopamin, Octopamin, Tyramin, 2-Phenylethylamin, Phenylethanolamin, Epinin (N-Methyldopamin), Synephrin, Ephedrin, Pseudoephedrin, Norephedrin und Isoprenalin.

Einige dieser Verbindungen wurden in der Literatur bereits auf ihre Aktivität hinsichtlich des beta-3-adrenergen-Rezeptors bei menschlichen Fettzellen sowie von Säugetieren untersucht (Naunyn-Schmiedeberg's Archives of Pharmacology 1999, 359, 310-321).

Bevorzugt sind Verbindungen der Formel (PhEA), bei denen R 17 = H ist und R16 Wasserstoff oder $C_1$-$C_4$-Alkyl, bevorzugt Wasserstoff, Methyl oder iso-Propyl, bedeutet. Ferner bevorzugt sind Verbindungen der Formel (PhEA), bei denen zusätzlich R15 = H ist.

In einer weiteren bevorzugten Ausgestaltung sind die Agonisten beta-adrenerger-Rezeptoren solche Verbindungen, bei denen R12 und R 17 = H ist.

Insbesondere bevorzugte Agonisten beta-adrenerger-Rezeptoren entsprechen der Formel (PhEA-i), dabei wiederum bevorzugt sind Tyramin, N-Methyltyramin, Octopamin, und Synephrin.

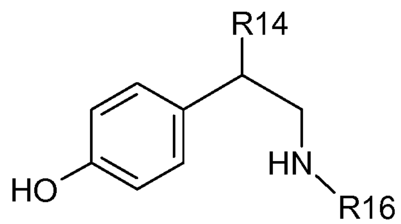

(PhEA-i)

wobei die Reste R14 und R16 die oben genannte (bevorzugte) Bedeutung haben.

Der am meisten bevorzugte Agonist eines beta-adrenergen-Rezeptors ist Synephrin (R14 = OH, R16 = $CH_3$ in Formel (PhEA-i)), vorzugsweise racemisch oder enantiomerenrein, dabei wiederum bevorzugt ist die (-)-Form. Ebenfalls besonders bevorzugt sind Synephrin-haltige Extrakte, wie beispielsweise Orangenblüten-Extrakt.

Besonders bevorzugt enthält eine kosmetische, vorzugsweise topische, erfindungsgemäß zu verwendende Zubereitung einen Agonisten eines beta-adrenergen-Rezeptors, dabei bevorzugt Synephrin, vorzugsweise in einer Gesamtmenge von 0,0001 - 0,10 Gew.-%, bevorzugt von 0,001 - 0,05 Gew.-%, weiter bevorzugt von 0,002 - 0,02 Gew.-%, jeweils bezogen auf das Gesamtgewicht der Zubereitung, wobei bei Salzen das Gegenion des Agonisten nicht mitgerechnet wird.

Bevorzugt sind die Gewichtsverhältnisse der Gesamtmenge der Verbindung der Formel (I) zu der Gesamtmenge an Agonisten eines beta-adrenergen-Rezeptors, insbesondere zu Synephrin, in den erfindungsgemäßen Zubereitungen gewählt von 100 : 1 bis 1 : 5, weiter bevorzugt von 50 : 1 bis 1 : 1, am meisten bevorzugt von 25 : 1 bis 2 : 1.

Da das Auftreten von Cellulite neben einer verstärkten Einlagerung von Fett im Fettgewebe zumeist auch mit einem Abbau des Bindegewebes einher geht, können erfindungsgemäß bevorzugt kosmetisch zu verwendende Zubereitungen enthaltend eine oder mehrere Verbindungen der Formel (I) auch Wirkstoffe enthalten, die einem Abbau des Bindegewebes vorbeugen. Vorteilhaft sind dabei Wirkstoffe, die Matrix-Metallo-Proteinasen (MMPs) hemmen. Diese Enzyme sind in der Lage, Makromoleküle der extrazellulären Matrix (ECM) / des Bindegewebes, zu der auch die Collagene zählen, proteolytisch abzubauen. Insbesondere die Matrix-Metallo-Proteinase-1 (MMP-1), Matrix-Metallo-Proteinase-2 (MMP-2) und Matrix-Metallo-Proteinase-9 (MMP-9) sind für den Abbau des Bindegewebes der Haut verantwortlich. Eine Inhibition von MMPs ist zum Beispiel durch den Zusatz von Ursolsäure, Retinylpalmitat, Propylgallat, Precocene, 6-Hydroxy-7-methoxy-2,2-dimethyl-1(2H)-benzopyran, 3,4-Dihydro-6-hydroxy-7-methoxy-2,2-dimethyl-1(2H)-benzopyran möglich. Auch ein Zusatz von Peptiden, die MMPs hemmen, zu erfindungsgemäß zu verwendenden Zubereitungen ist zur Hemmung von MMPs vorteilhaft. Auch Proteine oder Glycoproteine aus Soja sowie hydrolysierte Proteinen aus Reis, Erbse oder Lupine inhibieren MMPs und sind somit ein geeigneter Zusatz. Eine Kombination mit einem Pflanzenextrakt, der MMPs hemmt, ist ebenfalls von Vorteil. Zu nennen ist dabei beispielhaft ein Extrakt aus Shiitake-Pilzen. Vorteilhaft ist auch die Kombination mit Extrakten aus den Blättern der Familie Rosaceae, Unterfamilie Rosoideae. Ganz besonders vorteilhaft ist die Verwendung von Brombeerblatt-Extrakt, insbesondere wie beschrieben in WO 2005/123101 A1.

Im Rahmen der vorliegenden Erfindung bevorzugt in Kombination zu verwendende MMP-Inhibitoren sind Retinylpalmitat, Propylgallat, Precocene, 6-Hydroxy-7-methoxy-2,2-dimethyl-1(2H)-benzopyran, 3,4-Dihydro-6-hydroxy-7-methoxy-2,2-dimethyl-1(2H)-benzopyran, Benzamidin Hydroclorid, die Cysteinproteinase-Inhibitoren N-Ethylmaleimid und epsilon-Aminon-Capronsäure der Serinprotease-Inhibitor: Phenylmethylsulfonylfluorid, Collhibin (Fa. Pentapharm; INCI: Hydrolyzed Rice Protein), Oenotherol (Fa. Soliance; INCI: Propylene Glycol, Aqua, Oenothera biennis root extract, Ellagsäure und Ellagitannine, z.B. aus Granatapfel), Phosphoramidon Hinokitiol, EDTA, Galardin, EquiStat (Fa. Collaborative Group; Apfelfruchtextrakt, Sojasamenextrakt: Ursolsäure, Sojaisoflavone & Sojaproteine), Salbeiextrakte, MDI (Fa. Atrium; INCI: Glycosaminoglycans), Fermiskin (Fa. Silab/Mawi; INCI: Water and Lentinus Edodes Extract), Actimp 1.9.3. (Fa. Expanscience/ Rahn; INCI: Hydrolyzed Lupine Protein), Lipobelle Soyaglycone (Fa. Mibelle; INCI: Alcohol, Polysorbate 80, Lecithin and Soy Isoflavones), Extrakte aus grünem und schwarzem Tee sowie zahlreiche weitere Pflanzenextrakte, die in WO 02/069992 (siehe dort Tabellen 1-12) gelistet sind.

Um dem Abbau des Bindegewebes entgegenzuwirken, ist des Weiteren in erfindungsgemäß bevorzugt kosmetisch zu verwendenden Zubereitungen enthaltend eine oder mehrere Verbindungen der Formel (I) die Kombination mit Wirkstoffen vorteilhaft, die die Bildung von Collagen im Gewebe fördern. Häufig zur Steigerung der Collagensynthese eingesetzte Einzelstoffe sind zum Beispiel Wirkstoffe wie Ascorbinsäure und deren Derivate, Retinol sowie Abkömmlinge des Retinols oder Pflanzenextrakte wie zum Beispiel Extrakte aus Aloe- und Centella-Arten. Darüber hinaus zählen auch peptidische Stoffe und deren Derivate wie z.B. Carnitin, Carnosin, Kreatin, Matrikine Peptide (e.g. lysyl-threonyl-threonyl-lysyl-serine) sowie weitere peptidische Strukturen wie palmitoylierte Pentapeptide (z.B. Matrixyl/Fa. Sederma) oder das Oligopeptid mit dem Handelsnamen Vincipeptide (Fa. Vincience/Frankreich) zu häufig verwendeten, die Collagensynthese steigernden Wirkstoffen. Im weiteren finden Verbindungen wie Asiatsäure (Asiatic acid), Madecassic Säure, Madecassosid, Asiaticosid, Extrakte aus Centella asiatica, Niacinamid, Astaxanthin, Glucane z.B aus Hefen und Hafer, Sojaextrakte sowie Sojaisoflavone wie Genistein und Daidzein, Rutin, Chrysin, Morin, Betelnuss-Alkaloide, Forskolin, Betulinsäure, Extrakte aus Plantago Arten, TGF-beta, Extrakte aus Ginkgo biloba, Glutamin und Glycolsäure Einsatz als Collagensynthesestimulatoren. Besonders bevorzugt ist dabei der Zusatz einer Kombination aus Aloe vera Extrakt, Himbeerblatt-Extrakt und Magnesiumascorbylphosphat.

Desweiteren bevorzugt zu verwendende Zubereitungen enthaltend eine oder mehrere Verbindungen der Formel (I) umfassen ferner zusätzlich (i) Coenzym A zur Förderung des Transportes der freien Fettsäuren in den Mitochondrien und/oder (ii) L-Carnitin zur Förderung der beta-Oxidation.

Substanzen und Hilfsstoffe, welche eine erfindungsgemäß zu verwendende Zubereitung enthaltend eine oder mehrere Verbindungen der Formel (I) zusätzlich enthalten kann, sind beispielsweise:

Konservierungsmittel, vorzugsweise die in US 2006/0089413 genannten, Abrasiva, Antiakne-Mittel und Mittel zu Sebumreduktion, vorzugsweise die in WO 2008/046791 genannten, Mittel gegen Hautalterung, vorzugsweise die in WO 2005/123101 genannten, antibakterielle Mittel, Anticellulitis-Mittel, Antischuppen-Mittel, vorzugsweise die in WO 2008/046795 genannten, entzündungshemmende Mittel, irritationsverhindernde Mittel, Antiirritantien (antiinflammatorische, irritationshemmende und irritationsverhindernde Mittel), vorzugsweise die in WO 2007/042472 und US 2006/0089413 genannten, antimikrobielle Mittel, vorzugsweise die in WO 2005/123101 genannten, Antioxidantien, vorzugsweise die in WO 2005/123101 genannten, Adstringentien, antiseptische Mittel, Antistatika, Binder, Puffer, Trägermaterialien, vorzugsweise die in WO 2005/123101 genannten, Chelatbildnder, vorzugsweise die in WO 2005/123101 genannten, Zellstimulantien, reinigende Mittel, pflegende Mittel, Enthaarungsmittel, oberflächenaktive Substanzen, deodorierende Mittel und Antiperspirantien, vorzugsweise die in WO 2005/123101 genannten, Weichmacher, Emulgatoren, vorzugsweise die in WO 2005/123101 genannten, Enzyme, ätherische Öle, vorzugsweise die in US 2008/0070825 genannten, Insektenrepellentien, vorzugsweise die in WO 2005/123101 genannten, Fasern, Filmbildner, Fixateure, Schaumbildner, Schaumstabilisatoren, Substanzen zum Verhindern des Schäumens, Schaumbooster, Fungizide, gelierende Mittel und gelbildende Mittel, vorzugsweise die in WO 2005/123101 genannten, Haarpflegemittel, Haarverformungsmittel, Haarglättungsmittel, Feuchtigkeitsregulatoren (feuchtigkeitsspendende, anfeuchtende und/oder feuchthaltende Substanzen), vorzugsweise die in WO 2005/123101 genannten, Osmolyte, vorzugsweise die in WO 2005/123101 genannten, kompatible Solute, vorzugsweise die in WO 01/76572 und WO 02/15686 genannten, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, vorzugsweise die in WO 2008/046676 genannten, Pulver, Proteine und Proteinhydrolysate, vorzugsweise die in WO 2005/123101 und WO 2008/046676 genannten, rückfettende Mittel, abschleifende Mittel, hautberuhigende Mittel, hautreinigende Mittel, hautpflegende Mittel, hautheilende Mittel (skin repair agents), vorzugsweise enthaltend Cholesterin und/oder Fettsäuren und/oder Ceramide und/oder Pseudoceramide, dabei bevorzugt die in WO 2006/053912 genannten, Hautaufhellungsmittel, vorzugsweise die in WO 2007/110415 genannten, hautschützende Mittel, hauterweichende Mittel, hautkühlende Mittel, vorzugsweise die in WO 2005/123101 genannten, hautwärmende Mittel, vorzugsweise die in WO 2005/123101 genannten, Stabilisatoren, UV-absorbierende Mittel und UV-Filter, vorzugsweise die in WO 2005/123101 genannten, Benzyliden-beta-dicarbonylverbindungen, vorzugsweise die in WO 2005/107692 genannten, alpha-Benzoylzimtsäurenitrile, vorzugsweise die in WO 2006/015954 genannten, AhR-Rezeptor-Antagonisten, vorzugsweise die in WO 2007/128723 und WO 2007/060256 genannten, Waschmittel, Weichspüler, suspendierende Mittel, Hautbräunungsmittel, vorzugsweise die in WO 2006/045760 genannten, Verdickungsmittel, Vitamine, vorzugsweise die in WO 2005/123101 genannten, Öle, Wachse und Fette, vorzugsweise die in WO 2005/123101 genannten, Phospholipide, vorzugsweise die in WO 2005/123101 genannten, Fettsäuren (gesättigte Fettsäuren, ein- oder mehrfach ungesättigte Fettsäuren, $\alpha$-Hydroxysäuren, Polyhydroxyfettsäuren), vorzugsweise die in WO 2005/123101 genannten, Verflüssiger, Farbstoffe und farbschützende Mittel sowie Pigmente, vorzugsweise die in WO 2005/123101 genannten, Antikorrosiva, Aromen und Geschmackstoffe sowie Riechstoffe, vorzugsweise die in S. Arctander, Perfume and Flavor Chemicals, Eigenverlag, Montclair, N.J., 1969 und Surburg, Panten, Common Fragrance and Flavor Materials, 5th Edition, Wiley-VCH, Weinheim 2006 aufgeführten, insbesondere die in US 2008/0070825 explizit genannten, Alkohole und Polyole, vorzugsweise die in WO 2005/123101 genannten, Tenside, vorzugsweise die in WO 2005/123101 genannten, tierische Extrakte, Hefeextrakte, Extrakte von Algen oder Mikroalgen, Elektrolyte, Verflüssiger, organische Lösungsmittel, vorzugsweise die in WO 2005/123101 genannten, oder Silikone und Silikonderivate, vorzugsweise die in WO 2008/046676 genannten.

Ferner vorteilhaft sind erfindungsgemäß zu verwendende Zubereitungen, die oral verabreicht werden, z.B. in Form von Tabletten (z.B. Filmtabletten), Dragees, Kapseln (z.B. Gelatinekapseln), Granulaten, Säften, Lösungen, Emulsionen, Mikroemulsionen, Sprays oder in sonstiger Form oral konsumierbarer Produkte oder in Form von Lebensmitteln, welche auf Grund der darin enthaltenen Verbindung(en) der Formel (I) "Schönheit von Innen" ("beauty from inside") bewirken.

Bevorzugte kosmetische Trägerstoffe, die Bestandteil einer erfindungsgemäß zu verwendenden Zubereitung sein können, sind bei 25°C und 1013 mbar fest oder flüssig (inklusive hochviskoser Substanzen).

Bevorzugte flüssige Trägerstoffe, die Bestandteil einer erfindungsgemäß zu verwendenden Zubereitung sein können, sind ausgewählt aus der Gruppe bestehend aus Glycerin, 1,2-Propylenglycol, 1,2-Butylenglycol, 1,3-Butylenglycol, 1,2-Pentandiol, 1,2-Hexandiol, 1,2-Octandiol, 1,2-Decandiol, Ethanol, Wasser und Mischungen zweier oder mehrerer der genannten flüssige Trägerstoffe mit Wasser. Gegebenenfalls können diese erfindungsgemäß zu verwendenden Zubereitungen hergestellt werden unter Zusatz eines Konservierungsmittels, Lösungsvermittlers oder Antioxidans.

Bevorzugte feste Trägerstoffe, die Bestandteil einer erfindungsgemäß zu verwendenden Zubereitung sein können, sind Hydrokolloide wie Stärken, abgebaute Stärken, chemisch oder physikalisch modifizierte Stärken, Dextrine, (pulverförmige) Maltodextrine (bevorzugt mit einem Dextroseequivalent-Wert von 5 bis 25, bevorzugt von 10 - 20), Lactose, Siliciumdioxid, Glucose, modifizierte Cellulosen, Gummi Arabicum, Ghatti-Gum, Traganth, Karaya, Carrageenan, Pullulan, Curdlan, Xanthan Gum, Gellan Gum, Guarkernmehl, Johannisbrotkernmehl, Alginate, Agar, Pektin und Inulin sowie Mischungen zweier oder mehrerer dieser Feststoffe, insbesondere Maltodextrine (bevorzugt mit einem Dextroseequivalent-Wert von 15 - 20), Lactose, Siliciumdioxid und/oder Glucose.

Weiterhin können die erfindungsgemäß zu verwendenden Zubereitungen in verkapselter Form vorliegen, wobei diese vorzugsweise verkapselt sind mit einem festen Hüllmaterial, das vorzugsweise ausgewählt ist aus Stärken, abgebaute oder chemisch oder physikalisch modifizierte Stärken (insbesondere Dextrine und Maltodextrine), Gelatinen, Gummi Arabicum, Agar-Agar, Ghatti-Gummi, Gellan Gum, modifizierte und nicht modifizierte Cellulosen, Pullulan, Curdlan, Carrageenane, Alginsäure, Alginate, Pektin, Inulin, Xanthan Gum und Mischungen zweier oder mehrerer der genannten Substanzen.

Vorzugsweise wird das feste Hüllmaterial gewählt aus Gelatine (vorteilhaft sind Schweine-, Rinder-, Hühner- und/oder Fischgelatinen und Mischungen davon, vorzugsweise umfassend mindestens eine Gelatine mit einem Bloom-Wert von größer oder gleich 200, bevorzugt mit einem Bloom-Wert von größer oder gleich 240), Maltodextrin (vorzugsweise aus Mais, Weizen, Tapioka oder Kartoffel gewonnen, bevorzugte Maltodextrine weisen einen DE-Wert von 10 - 20 auf), modifizierte Cellulose (z.B. Celluloseether), Alginate (z.B. Na-Alginat), Carrageenan (beta-, iota-, lambda- und/oder kappa- Carrageenan), Gummi Arabicum, Curdlan und/oder Agar-Agar. Gelatine wird insbesondere wegen ihrer guten Verfügbarkeit in unterschiedlichen Bloom-Werten bevorzugt verwendet. Besonders bevorzugt namentlich zur oralen Verwendung sind nahtlose Gelatine- oder Alginatkapseln, deren Hülle sich im Mund sehr schnell auflöst bzw. beim Kauen zerplatzt. Die Herstellung kann beispielsweise erfolgen wie beschrieben in EP 0 389 700, US 4,251,195, US 6,214,376, WO 03/055587 oder WO 2004/050069.

Wesentliche Anwendungsgebiete für die erfindungsgemäßzu verwendenden Zubereitungen sind kosmetische, insbesondere dermatologische Zubereitungen, die (abgesehen von der oder den Verbindung(en) der Formel (I)) wie üblich zusammengesetzt sind und dem kosmetischen, insbesondere dermatologischen Lichtschutz, zur Behandlung, der Pflege und der Reinigung der Haut und/oder der Haare oder als Schminkprodukt in der dekorativen Kosmetik dienen. Entsprechend können derartige Zubereitungen, je nach ihrem Aufbau beispielsweise als Hautschutzcreme, Tages- oder Nachtcreme, Augencreme, Sonnenschutz- oder After-sun-Lotion, Nährcreme, Pflegemaske, Gel-Pads, Gesichtswasser, feuchte Pflege- und Reinigungstücher, Reinigungsmilch, Reinigungsseife, Schaum- oder Duschbad, Deodorant, Antitranspirant, Haarshampoo, Haarpflegemittel, Haarconditioner, Haarcoloration, Haarstylingmittel verwendet werden und dabei bevorzugt als Emulsion, Lotion, Milch, Fluid, Creme, Hydrodispersionsgel, Balm, Spray, alkoholische oder wässrig/alkoholische Lösung, Schaum, Puder, Flüssigseife, Seifenstück, Shampoo, Roll-on, Stick oder Make-up vorliegen. In Haarbehandlungsmitteln richtet sich die Verwendung vorzugsweise auf den Haarboden oder die Kopfhaut.

Der eine bzw. die mehreren weiteren Stoffe mit physiologischer Kühlwirkung, die als Bestandteil in einer erfindungsgemäß zu verwendenden Mischung eingesetzt werden können, werden dabei vorzugsweise ausgewählt aus der folgenden Liste: Menthol und Mentholderivate (z.B. L-Menthol, D-Menthol, racemisches Menthol, Isomenthol, Neoisomenthol, Neomenthol) Menthylether (z.B. (l-Menthoxy)-1,2-propandiol, (l-Menthoxy)-2-methyl-1,2-propandiol, l-Menthyl-methylether), Menthylester (z.B. Menthylformiat, Menthylacetat, Menthylisobutyrat, Menthyllactate, L-Menthyl-L-lactat, L-Menthyl-D-lactat, Menthyl-(2-methoxy)acetat, Menthyl-(2-methoxyethoxy)acetat, Menthylpyroglutamat), Menthylcarbonate (z.B. Menthylpropylenglycolcarbonat, Menthylethylenglycolcarbonat, Menthylglycerincarbonat oder deren Mischungen), die Halbester von Mentholen mit einer Dicarbonsäure oder deren Derivate (z.B. Mono-Menthylsuccinat, Mono-Menthylglutarat, Mono-Menthylmalonat, O-Menthyl-bernsteinsäureester-N,N-(dimethyl)amid, O-Menthylbernsteinsäureesteramid), Menthancarbonsäureamide (dabei vorzugsweise Menthancarbonsäure-N-ethylamid [WS3] oder $N^{\alpha}$-(Menthancarbonyl)glycinethylester [WS5], wie in US 4,150,052 beschrieben, Menthancarbonsäure-N-(4-cyanophenyl)amid oder Menthancarbonsäure-N-(4-cyanomethylphenyl)amid wie in WO 2005/049553 beschrieben, Menthancarbonsäure-N-(alkoxyalkyl)amide), Menthon und Menthonderivate (z.B. L-Menthonglycerinketal), 2,3-Dimethyl-2-(2-propyl)-butansäurederivate (z.B. 2,3-Dimethyl-2-(2-propyl)-butansäure-N-methylamid [WS23]), Isopulegol oder seine Ester (l-(-)-Isopulegol, l-(-)-Isopulegolacetat), Menthanderivate (z.B. p-Menthan-3,8-diol), Cubebol oder synthetische oder natürliche Mischungen, enthaltend Cubebol, Pyrrolidonderivate von Cycloalkyldionderivaten (z.B. 3-Methyl-2(1-pyrrolidinyl)-2-cyclopenten-1-on) oder Tetrahydropyrimidin-2-one (z.B. Icilin oder verwandte Verbindungen, wie in WO 2004/026840 beschrieben).

Der oder die mehreren weiteren Stoffe mit physiologischer Kühlwirkung, die als Bestandteil einer erfindungsgemäß zu verwendenden Mischung eingesetzt werden können, sind insbesondere vorzugsweise Stoffe, welche zumindest im Wesentlichen eine physiologische Kühlwirkung verursachen. Solche bevorzugten Stoffe sind: Menthylether (z.B. (l-Menthoxy)-1,2-propandiol, (l-Menthoxy)-2-methyl-1,2-propandiol), polarere Menthylester (z.B. Menthyllactate, L-Menthyl-L-lactat, L-Menthyl-D-lactat, Menthyl-(2-methoxy)acetat, Menthyl-(2-methoxyethoxy)acetat, Menthylpyroglutamat), Menthylcarbonate (z.B. Menthylpropylenglycolcarbonat, Menthylethylenglycolcarbonat, Menthylglycerincarbonat), die Halbester von Mentholen mit einer Dicarbonsäure oder deren Derivate (z.B. Mono-Menthylsuccinat, Mono-Menthylglutarat, Mono-Menthylmalonat, O-Menthylbernsteinsäureester-N,N-(dimethyl)amid, O-Menthylbernsteinsäureesteramid), nicht erfindungsgemäße Menthancarbonsäureamide (z.B.Menthancarbonsäure-N-ethylamid [WS3], $N^{\alpha}$-(Menthancarbonyl)glycinethylester [WS5], Menthancarbonsäure-N-(4-cyanophenyl)amid, Menthancarbonsäure-N-(alkoxyalkyl)amide), Menthonderivate (z.B. L-Menthonglycerinketal), 2,3-Dimethyl-2-(2-propyl)-butansäurederivate (z.B. 2,3-Dimethyl-2-(2-propyl)-butansäure-N-methylamid), Pyrrolidonderivate von Cycloalkyldionderivaten (z.B. 3-Methyl-2(1-pyrrolidinyl)-2-cyclopenten-1-on) oder Tetrahydropyrimidin-2-one (z.B. Icilin oder verwandte Verbindungen, die in WO 2004/026840 beschrieben sind).

Komponenten, die ein Wärme-, Schärfe-, Kribbel- oder Prickelgefühl auf der Haut oder auf den Schleimhäuten hervorrufen, insbesondere Aromastoffe mit einem wärmeerzeugenden Effekt und/oder scharf schmeckende Verbindungen (Scharfstoffe), die neben einer oder mehreren Verbindungen der Formel (I) Bestandteil einer erfindungsgemäß zu

verwendenden Zubereitung sein können, sind in WO 2005/123101 genannt.

Zur Anwendung in der für Kosmetika und Dermatika üblichen Weise werden die Verbindungen der Formel (I) auf die Haut und/oder die Haare in ausreichender Menge aufgebracht.

Besondere Vorteile bieten dabei kosmetische und dermatologische Zubereitungen, die eine oder mehrere Verbindungen der Formel (I) enthalten und zusätzlich als Sonnenschutzmittel wirken. Vorteilhaft enthalten diese Zubereitungen mindestens einen UVA-Filter und/oder mindestens einen UVB-Filter und/oder mindestens ein anorganisches Pigment. Die Zubereitungen können dabei in verschiedenen Formen vorliegen, wie sie z.B. üblicherweise für Sonnenschutzzubereitungen eingesetzt werden. So können sie z.B. eine Lösung, eine Emulsion vom Typ Wasser-in-Öl (W/O) oder vom Typ Öl-in-Wasser (O/W), oder eine multiple Emulsion, beispielsweise vom Typ Wasser-in-Öl-in-Wasser (W/O/W), ein Gel, eine Hydrodispersion, einen festen Stift oder auch ein Aerosol bilden.

Erfindungsgemäß zu vewendende Zubereitungen im Bereich Kosmetika und Dermatika, die eine oder mehrere Verbindungen der Formel (I) enthalten, werden besonders vorteilhaft mit Substanzen kombiniert, die UV-Strahlung absorbieren oder reflektieren, namentlich für kosmetische oder hautschützende Zwecke (also nicht für mundhygienische Zwecke), wobei die Gesamtmenge der Filtersubstanzen von 0,01 Gew.-% bis 40 Gew.-%, vorzugsweise 0,1% bis 10 Gew.-%, insbesondere 1,0 bis 5,0 Gew.-% beträgt, bezogen auf das Gesamtgewicht der Zubereitungen, um kosmetische Zubereitungen zur Verfügung zu stellen, die das Haar bzw. die Haut vor ultravioletter Strahlung schützen. Vorteilhaft enthalten diese Zubereitungen mindestens einen UVA-Filter und/oder mindestens einen UVB-Filter und/oder mindestens ein anorganisches Pigment, so dass ein Lichtschutzfaktor von zumindest > 2 (bevorzugt > 5) erreicht wird. Diese erfindungsgemäß zu verwendenden Zubereitungen können dabei in verschiedenen Formen vorliegen, wie sie z.B. üblicherweise für Sonnenschutzzubereitungen eingesetzt werden. So können sie z.B. eine Lösung, eine Emulsion vom Typ Wasser-in-Öl (W/O) oder vom Typ Öl-in-Wasser (O/W), oder eine multiple Emulsion, beispielsweise vom Typ Wasser-in-Öl-in-Wasser (W/O/W), ein Gel, eine Hydrodispersion, einen festen Stift oder auch ein Aerosol darstellen.

Vorteilhafte UV-Filter und anorganische Lichtschutzpigmente sind genannt in WO 2005/123101. Besonders zur Kombination geeignete UV-Absorber sind ebenfalls genannt in WO 2005/123101.

Vorteilhafte anorganische Lichtschutzpigmente sind feindisperse Metalloxide und Metallsalze die ebenfalls in WO 2005/123101 genannt sind. Die Gesamtmenge an anorganischen Pigmenten, insbesondere hydrophoben anorganischen Mikropigmenten in den fertigen kosmetischen Zubereitungen liegt vorteilhaft von 0,1 bis 30 Gew.-%, bevorzugt 0,5 bis 10,0, bezogen auf das Gesamtgewicht der Zubereitungen.

In manchen Zubereitungen kann auch eine Kombination mit (Metall)-Chelatoren vorteilhaft sein. Bevorzugt einzusetzende (Metall)-Chelatoren sind die in WO 2005/123101 genannten Verbindungen.

Erfindungsgemäß bevorzugt kosmetisch zu verwendende Zubereitungen können auch entzündungshemmende und/oder rötungs- und/oder juckreizlindernde Wirkstoffe enthalten. Vorteilhaft werden die in WO 2005/123101 genannten Verbindungen als entzündungshemmende bzw. rötungs- und/oder juckreizlindernde Wirkstoffe verwendet.

Die Menge der Antiirritantien (eine oder mehrere Verbindungen) in den erfindungsgemäß zu verwendenden Zubereitungen beträgt vorzugsweise 0,0001 bis 20 Gew.-%, besonders bevorzugt 0,0001 - 10 Gew.-%, insbesondere 0,001 - 5 Gew.-%, bezogen auf das Gesamtgewicht der Zubereitung.

Die eine oder mehrere Verbindungen der Formel (I) können insbesondere in kosmetischen und dermatologischen Zubereitungen vorteilhaft in Kombination mit Insektrepellents wie z.B. DEET, IR 3225, Dragorepel (Symrise GmbH & Co. KG) eingesetzt werden.

Die eine oder mehrere Verbindungen der Formel (I) können insbesondere in kosmetischen und dermatologischen Zubereitungen vorteilhaft in Kombination mit Haarpflegemitteln und Antischuppenwirkstoffen (z.B. Climbazol, Ketoconazol, Piroctonoleamin, Zink-Pyrithion) eingesetzt werden.

Auch können die Verbindungen der Formel (I) in zahlreichen Fällen vorteilhaft in Kombination mit einem oder mehreren Konservierungsmitteln in erfindungsgemäß zu verwendenden Zubereitungen eingesetzt werden. Vorzugsweise gewählt werden hierbei die in WO 2005/123101 genannten Konservierungsmittel.

Erfindungsgemäß zu verwendende Zubereitungen können neben einer oder mehreren Verbindungen der Formel (I) auch für kosmetische Zwecke verwendbaren Pflanzenextrakte enthalten. Vorzugsweise werden die Pflanzenextrakte gewählt aus der auf Seite 44 der 3. Auflage des Leitfadens zur Inhaltsstoffdeklaration kosmetischer Mittel, herausgegeben vom Industrieverband Körperpflegemittel und Waschmittel e.V. (IKW), Frankfurt, beginnenden Tabelle aufgeführten Stoffe. Vorteilhaft sind ferner insbesondere die in WO 2005/123101 genannten Extrakte.

Kosmetische zu verwendende Zubereitungen, enthaltend eine oder mehrere Verbindungen der Formel (I), können, insbesondere wenn kristalline oder mikrokristalline Festkörper wie beispielsweise anorganische Mikropigmente in die Zubereitungen eingearbeitet werden sollen, erfindungsgemäß auch in WO 2005/123101 genannte anionische, kationische, nichtionische und/oder amphotere Tenside enthalten.

Die oberflächenaktive Substanz kann in einer Konzentration zwischen 1 und 98 Gew.-% in den erfindungsgemäß zu verwendenden Zubereitungen vorliegen, bezogen auf das Gesamtgewicht der Zubereitungen.

Die Ölphase erfindungsgemäß zu verwendender Zubereitungen, die eine oder mehrere Verbindungen der Formel (I) enthalten, kann vorteilhaft gewählt werden aus den in WO 2005/123101 genannten Substanzgruppen.

Im Folgenden wird die Erfindung anhand von Beispielen weiter erläutert. Die Beispiele dienen zur Verdeutlichung der Erfindung, ohne den Schutzbereich der Patentansprüche einzuschränken. Sofern nicht anders angegeben, beziehen sich alle Angaben auf das Gewicht.

Die in den im Folgenden beschriebenen Assays eingesetzten Verbindungen der Formel (I) wurden entweder in reiner Form synthetisiert (Verbindung (B)) oder aus Pao Rosa-Kernholz Extrakt isoliert (enthaltend Maackiain und Medicarpin).

**Beispiel 1: Herstellung von 3-Methoxypterocarpan Verbindung (B)**

Beispiel 1.1: 1-(2,4-Dimethoxyphenyl)-2-(2'-methoxyphenyl)-1-ethanon

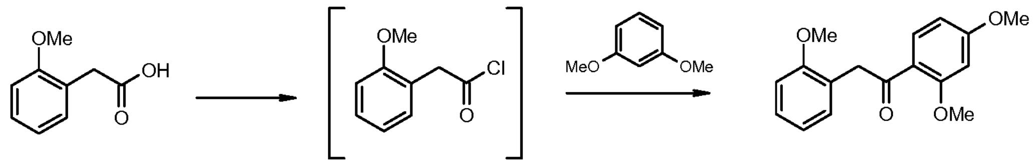

66 g (0,55 mol) Thionylchlorid werden unter $N_2$-Atmosphäre und Zusatz von 0,4 g Harnstoff vorgelegt, anschließend gibt man unter Rühren 66 g (0,40 mol) 2-Methoxyphenylessigsäure in mehreren Portionen innerhalb von 30 min zu. Nach 2 h Rückfluss wird mit 90 g Dichlormethan versetzt und diese Lösung in eine Mischung aus 69 g (0,50 mol) Resorcindimethylether und 67 g (0,50 mol) $AlCl_3$ in 300 g Dichlormethan innerhalb 1 h bei RT (Raumtemperatur; ca. 20°C) dosiert. Man rührt noch 2 h nach und hydrolysiert in 500 g Eiswasser. Es wird mit Wasser und Na-bicarbonat neutral gewaschen, das Dichlormethan abdestilliert und der Rückstand aus Toluol kristallisiert. Ausbeute: 177 g (60% d.Th.)

**Beispiel** 1.2: 1-(2,4-Dimethoxyphenyl)-2-(2'-methoxyphenyl)-2-hydroxymethylen-1-ethanon

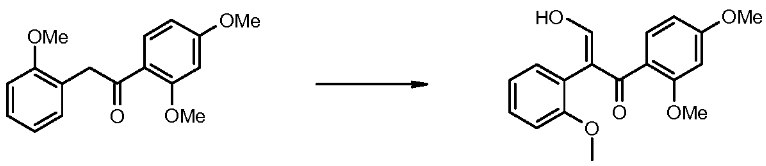

4,2 g (78 mmol) Na-methylat werden in 300 g Methyl-tert.-butylether (MTBE) unter $N_2$-Atmosphäre und Rühren vorgelegt. Bei 5°C wird eine Lösung aus 177 g (0,62 mol) des Produkt aus Beispiel 1.1 und 50 g (0,83 mol) Methylformiat in 180 g Tetrahydrofuran innerhalb 30 min zudosiert. Anschließend gibt man 30 g (0,56 mol) Na-methylat zu und rührt 16 h bei RT. Nach Hydrolyse mit 800 g Wasser unter Kühlung und weiterer Zugabe von 100 g MTBE trennt man die Phasen. Die wässrige Phase wird mit 10%iger Salzsäure auf pH 5-6 gestellt und mit 400 g MTBE extrahiert. Nach Einengen liegen 150 g Produkt vor. Ausbeute: 150 g (79% d.Th.)

Beispiel 1.3: 2'-Hydroxy-7-methoxy-isoflavon

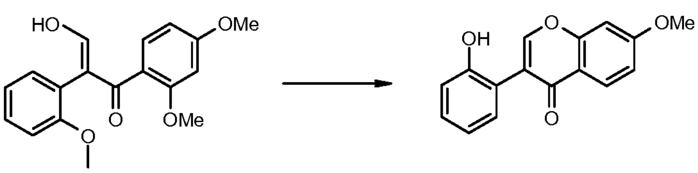

24 g (76 mmol) Produkt aus Beispiel 1.2 werden in 100 g Dichlormethan unter Rühren und $N_2$-Atmosphäre

vorgelegt und man dosiert innerhalb von 300 g (0,3 mol) einer 1M Lösung von Bortrichlorid in Dichlormethan. Es wird 18 h bei 30 - 40°C nachgerührt, mit 300 g Wasser unter Kühlung hydrolisiert. Nach Phasentrennung wird die organische Phase neutral gewaschen und nach Abziehen des Lösungsmittels der Rückstand aus Ethanol umkristallisiert. Ausbeute: 8 g (40% d.Th.)

Beispiel 1.4: 3-Methyoxypterocarpan (Verbindung (B))

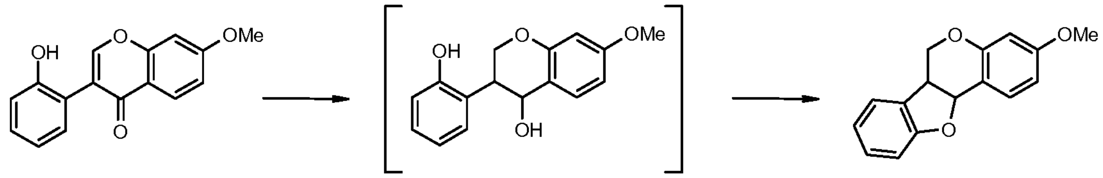

45 g (0,17 Mol) des Produktes aus Beispiel 1.3 werden in 1000 g Ethanol unter Rühren gelöst, portionsweise mit 64 g (0,1 mol) Na-borhydrid bei RT versetzt und 4 h gerührt. Dann wird das Lösungsmittel bei einer Sumpftemperatur von 50°C unter Vakuum entfernt, der verbleibende Rückstand mit 500 g MTBE und mit 500 g Wasser versetzt, mit 10%iger Salzsäure neutral gestellt und anschließend die Phasen getrennt. Zu dieser Lösung gibt man 1,2 g Bortrifluorid-Etherat (48%ig / 5 mmol) bei 0°C, rührt 2 h bei dieser Temperatur und weitere 3 h bei 40°C nach. Man wäscht mit Wasser und Na-bicarbonat neutral, engt die organische Phase auf ca. 150 g ein und kühlt auf RT ab, wonach das Produkt als weisses Kristallisat ausfällt. Nach Trocknung und Umkristallisation liegen 26 g (60% d.Th.) des Produktes der Formel (B) vor.

**Beispiel 2: Herstellung Pao Rosa Kernholz Extrakt**

Ein Trockenextrakt aus Pao Rosa-Kernholz enthaltend Verbindungen der Formel (I), insbesondere die Verbindungen Maackiain und Medicarpin, wird hergestellt. Als Trockenextrakt ist ein Extrakt hergestellt nach dem nachfolgenden Verfahren nach vollständiger Entfernung des Extraktionsmittels anzusehen. Das Verfahren zum Herstellen eines Trockenextraktes aus Pao Rosa-Kernholz umfasst folgende Schritte:

a) Versetzen von Holzraspat hergestellt aus abgelagertem Pao Rosa-Kernholz mit der 5-fachen Masse an Extraktionsmittel (eine Mischung aus Wasser und Ethanol mit einem Anteil an Ethanol von 30 %),
b) Extrahieren des Holzraspates mit dem Extraktionsmittel für die Dauer von 2 - 4 h, wobei eine Extraktionstemperatur von 80 - 100 °C eingehalten wurde.
c) Behandlung des Rohextraktes mit Aktivkohle, wobei das Gewichtsverhältnis von Aktivkohle zu in Punkt a) eingesetztem Holzraspat bei 10:1 betrug.

Charakterisierung der Extrakte erfolgt durch HPLC-Fingerprint-Analyse und quantitative Bestimmung des Gehaltes der Isoflavonoide sowie Pterocarpane: Säule: YMC ODS-AQ, 5 μm, 150x3 mm mit Vorsäule, Temperatur: 40°C, Fluß: 0,6 ml/min, Acetonitril/Wasser mit 0.1% Ameisensäure-Gradient, Injektionsvolumen: 5 μl, Detektionswellenlänge: 232 nm.

Figur 1 zeigt das erhaltene HPLC-Chromatogramm für einen Pao Rosa Rohextrakt vor der Behandlung mit Aktivkohle.

Figur 2 zeigt das erhaltene HPLC-Chromatogramm für einen Pao Rosa Trockenextrakt nach der Behandlung mit Aktivkohle.

Durch die Behandlung des Rohextraktes mit Aktivkohle in Schritt c) nimmt der Gehalt der Verbindungen der Formel (I), insbesondere der Verbindungen Maackiain und Medicarpin, zu. Dagegen wird der Gehalt des Isoflavons Afrormosin, das für ein hohes phototoxisches Potential bekannt ist, reduziert.

Tab. 2.1: Beispiel für den Gehalt der Pterocarpane Maackiain und Medicarpin sowie dem Isoflavon Afformosin in einem Pao Rosa Kernholz Extrakt vor sowie nach der Behandlung mit Aktivkohle, angegeben ist der Gehalt bezogen auf den Trockensubstanzgehalt des entsprechenden Extraktes.

| **Retentionszeit** | **Substanz** | **HPLC Gehalt** | |
|---|---|---|---|
| | | **Extraktion bis inkl. b)** | **Extraktion bis inkl. c)** |
| 5,3 min | Di-Hydroxy-Dimethoxy Isoflavon MG: 314 Odoratin | 3,8 % | 4,5 % |
| 5,7 min | Di-Hydroxy-Trimethoxy Isoflavon MG: 344 | 4,7 % | 4,8 % |
| 8,0 min | Isoflavon MG: 328 | 3,9 % | 3,3 % |
| 8,4 min | **2',7-Dihydroxy-4',6-dimethoxyisoflavon (MG:314)** | 5,8 % | 5,8 % |
| 10,3 min | **Afrormosin MG:298** | 8,4 % | 7,0 % |
| 11,1 min | **Maackiain MG:284** | 9,7 % | 11,8 % |
| 12,0 min | **Medicarpin MG:270** | 2,1 % | 2,5 % |

Der Extrakt sollte für die Verwendung in kosmetischen Zubereitungen frei von Extraktionsmittel sein. Das Extraktionsmittel sollte durch ein geeignetes Verfahren (z.B. Destillation, Trocknung, Gefriertrocknung) möglichst vollständig entfernt werden.

**Beispiel 3: Adipogenese-Assay (*in vitro*)**

3T3-L1-Zellen (Maus embryonale Fibroblasten-ähnliche Adipozyten-Zelllinie) werden in eine 48-well-Platte mit Collagen I-Beschichtung in einer Konzentration von 3 x $10^4$ Zellen / Well ausgesät. Nach 72 h Kultivierung bei 37 °C und 5% $CO_2$ in DMEM (Dulbecco's Modified Eagle Medium), angereichert mit 10% Kälberserum, werden verschiedene Konzentrationen der Testsubstanzen in DMEM, angereichert mit 10% fötalem Kälberserum sowie versetzt mit 1 μg/ml Insulin, 0,25 μM Dexamethason und 0,5 mM IBMX (3-Isobutyl-1-Methylxanthin), zugegeben und weitere 48 h inkubiert. Es erfolgt ein Medienwechsel, indem DMEM, angereichert mit 10% fötalem Kälberserum sowie versetzt mit 1 μg/ml Insulin, aufgetragen wird. Nach erneuter Kultivierung für 48 h erfolgt ein weiterer Medienwechsel, bei dem DMEM, angereichert mit 10% fötalem Kälberserum, aufgetragen wird.

Nach weiterer Inkubation für 72 h erfolgt die Quantifizierung der intrazellulär eingelagerten Lipide als Maß für die Differenzierung der Zellen durch Messung der Fluoreszenz nach Anfärbung der Lipide mit dem Fluoreszenz-Farbstoff Nilrot (Nile Red).

Die Inhibition der Adipogenese in Anwesenheit der Testsubstanzen wird gemäß der folgenden Gleichung berechnet:

$$\text{Inhibition der Adipogenese } [\%] = 100 - \left( \frac{\text{RFU Testsubstanz} - \text{RFU Kontrolle ohne Zellen}}{\text{RFU Kontrolle} - \text{RFU Kontrolle ohne Zellen}} \times 100 \right)$$

mit

RFU Testsubstanz = relative Fluoreszenz-Einheiten der Wells mit Testsubstanz und mit Zellen

RFU Kontrolle = relative Fluoreszenz-Einheiten der Wells ohne Testsubstanz, aber mit Zellen

RFU Kontrolle ohne Zellen = relative Fluoreszenz-Einheiten der Wells ohne Testsubstanz und ohne Zellen

Aus der Adipogenese-Inhibition [%] in einer Reihe von Verdünnungen von getesteten Proben wird der $IC_{50}$ berechnet. Dies ist die Konzentration, bei der die Adipogenese zu 50 % gehemmt wird.

Tabelle 3.1: Adipogenese-Inhibition der Einzelsubstanzen (Mittelwerte aus mindestens 2 unabhängigen Versuchen)

| Testsubstanz | $IC_{50}$ (ppm) |
|---|---|
| 3-Methoxypterocarpan | 50,9 |
| Medicarpin | 54,0 |
| Maackiain | 25,5 |
| Pao Rosa Kernholz Extrakt | 4,7 |

**Beispiel 4: Lipogenese-Assay (*in vitro*)**

Unter Lipogenese wird die Einlagerung von Triglyceriden in Adipozyten verstanden. Die Hemmung dieser Einlagerung kann durch die Hemmung der Aktivität extrazellulärer Lipoprotein-Lipase (LPL) erfolgen, indem die Hydrolyse extrazellulärer Triglyceride und dadurch die Aufnahme freier Fettsäuren durch Adipozyten reduziert wird. Als Vorversuch wird die Hemmung von pankreatischer Lipase (PL) untersucht.

PL (Sigma-Aldrich) wird in Anwesenheit von Prüfsubstanzen in unterschiedlichen Einsatzkonzentrationen mit Methylumbelliferyl-Oleat (MUF-Oleat) als Substrat versetzt. Durch Hydrolyse des MUF-Oleats durch PL entsteht fluoreszierendes Methylumbelliferon (MUF), das quantifiziert wird. Die Hemmung der Hydrolyse des MUF-Oleats ist ein Maß für die Hemmung der Aktivität der PL.

$$\text{Inhibition der PL}\,[\%] = 100 - \left(\frac{\text{MUF Testsubstanz} - \text{MUF Kontrolle ohne PL}}{\text{MUF Kontrolle} - \text{MUF Kontrolle ohne PL}} \times 100\right)$$

mit

MUF Testsubstanz = MUF-Konzentration der Wells mit Testsubstanz und mit PL
MUF Kontrolle = MUF-Konzentration der Wells ohne Testsubstanz, aber mit PL
MUF Kontrolle ohne PL = MUF-Konzentration der Wells ohne Testsubstanz und ohne PL

Aus der Inhibition der PL [%] in einer Reihe von Verdünnungen von getesteten Proben wird der $IC_{50}$ berechnet. Dies ist die Konzentration, bei der die Aktivität der PL zu 50 % gehemmt wird.

Tabelle 4.1: Inhibition der PL durch die Einzelsubstanzen (Mittelwerte aus mindestens 2 unabhängigen Versuchen)

| Testsubstanz | $IC_{50}$ (ppm) |
|---|---|
| 3-Methoxypterocarpan | 350 |
| Medicarpin | 425 |
| Maackiain | 147 |
| Pao Rosa Kernholz Extrakt | 80 |

Die Ergebnisse zur Hemmung der PL dienen als Vorversuch und werden an LPL bestätigt. Zur Gewinnung von LPL werden 3T3-L1-Zellen (Maus embryonale Fibroblasten-ähnliche Adipozyten-Zelllinie) in eine 6-well-Platte mit Collagen I -Beschichtung in einer Konzentration von 3 x $10^5$ Zellen / Well ausgesät. Die Kultivierung und Differenzierung der Zellen erfolgt analog zu den Angaben in Beispiel 2 (Adipogenese-Assay). Während der Differenzierung wird verstärkt LPL exprimiert. Die LPL liegt Membran-gebunden vor und wird durch eine 1-stündige Inkubation mit Heparin-Lösung bei 2 - 8 °C in den Überstand der Zellen freigesetzt.

Die so gewonnene LPL wird in Anwesenheit von Prüfsubstanzen in unterschiedlichen Einsatzkonzentrationen mit Methylumbelliferyl-Oleat (MUF-Oleat) als Substrat versetzt. Durch Hydrolyse des MUF-Oleats durch LPL entsteht fluoreszierendes Methylumbelliferon (MUF), das quantifiziert wird. Die Hemmung der Hydrolyse des MUF-Oleats ist ein Maß für die Hemmung der Aktivität der LPL und somit der Lipogenese.

$$\text{Inhibition der LPL } [\%] = 100 - \left( \frac{\text{MUF Testsubstanz} - \text{MUF Kontrolle ohne LPL}}{\text{MUF Kontrolle} - \text{MUF Kontrolle ohne LPL}} \times 100 \right)$$

mit

MUF Testsubstanz = MUF-Konzentration der Wells mit Testsubstanz und mit LPL
MUF Kontrolle = MUF-Konzentration der Wells ohne Testsubstanz, aber mit LPL
MUF Kontrolle ohne LPL = MUF-Konzentration der Wells ohne Testsubstanz und ohne LPL

Aus der Inhibition der LPL [%] in einer Reihe von Verdünnungen von getesteten Proben wird der $IC_{50}$ berechnet. Dies ist die Konzentration, bei der die Aktivität der LPL und somit die Lipogenese zu 50 % gehemmt wird.

Tabelle 4.2: Inhibition der LPL durch die Einzelsubstanzen (Mittelwerte aus mindestens 2 unabhängigen Versuchen)

| **Testsubstanz** | **$IC_{50}$ (ppm)** |
|---|---|
| 3-Methoxypterocarpan | 67,0 |
| Medicarpin | 788 |
| Maackiain | 104,2 |
| Pao Rosa Kernholz Extrakt | 41,5 |

**Beispiel 5: Lipolyse-Assay (*In vitro*)**

Primäre humane subkutane Preadipozyten (Lonza) werden in eine 96-well Mikrotiterplatte in einer Konzentration von 1 x $10^4$ Zellen / Well ausgesät. Bis zum Erreichen der Konfluenz erfolgt eine Kultivierung bei 37 °C und 5% $CO_2$ in DMEM, angereichert mit 10% fötalem Kälberserum. Zur Induzierung der Differenzierung von Preadipozyten zu Adipozyten folgt die Zugabe von DMEM, angereichert mit 10% fötalem Kälberserum sowie versetzt mit 10 µg/ml Insulin, 1 µM Dexamethason und 1 mM IBMX (3-Isobutyl-1-Methylxanthin). Eine Kultivierung für 14 bis 20 Tage folgt. In dieser Zeit kann das Medium gegebenenfalls durch DMEM, angereichert mit 10% fötalem Kälberserum, ersetzt werden.

Verschiedene Konzentrationen der Testsubstanzen werden in DMEM, versetzt mit Bovinem Serumalbumin, auf die Zellen aufgetragen. Nach etwa 20-stündiger Inkubation erfolgt die Quantifizierung von freiem Glycerin im Überstand der Zellen, das nach Hydrolyse von Triglyceriden in den Zellen von diesen abgegeben wird und ein Maß für die Lipolyse der Zellen ist. Die Quantifizierung des freien Glycerins wird basierend auf einer enzymatischen Methode mit einem Free Glycerol Reagent vorgenommen.

Die Stimulation der Lipolyse in Anwesenheit der Testsubstanzen wird gemäß der folgenden Gleichung berechnet:

$$\text{Stimulation der Lipolyse } [\%] = \left( \frac{\text{A Testsubstanz} - \text{A Testsubstanz ohne Zellen}}{\text{A Kontrolle} - \text{A Kontrolle ohne Zellen}} \times 100 \right) - 100$$

mit

A Testsubstanz = Absorption der Wells mit Testsubstanz und mit Zellen
A Testsubstanz ohne Zellen = Absorption der Wells mit Testsubstanz ohne Zellen (Absorptionskontrolle)
A Kontrolle = Absorption der Wells ohne Testsubstanz, aber mit Zellen
A Kontrolle ohne Zellen = Absorption der Wells ohne Testsubstanz und ohne Zellen

Aus der Lipolyse-Stimulation [%] in einer Reihe von Verdünnungen von getesteten Proben wird der $EC_{50}$ berechnet. Dies ist die Konzentration, bei der die Lipolyse zu 50 % stimuliert wird.

Tabelle 5.1: Aktivität durch die Einzelsubstanzen bezogen auf die Lipolyse (Mittelwerte aus mindestens 2 unabhängigen Versuchen). Die Testsubstanzen zeigen keine Aktivität hinsichtlich Lipolyse bei den aufgeführten maximalen Einsatzkonzentrationen, das heisst durch die Testsubstanzen wird die Lipolyse weder inhibiert noch stimuliert.

| **Testsubstanz** | **Aktivität (max. Einsatzkonzentration (ppm))** |
|---|---|
| 3-Methoxypterocarpan | keine Aktivität (25 ppm) |
| Medicarpin | keine Aktivität (25 ppm) |
| Maackiain | keine Aktivität (25 ppm) |
| Pao Rosa Kernholz Extrakt | keine Aktivität (10 ppm) |

Die Inhibition der Lipolyse in Anwesenheit der Testsubstanzen wird gemäß der folgenden Gleichung berechnet:

$$\text{Inhibition der Lipolyse}[\%] = 100 - \left( \frac{\text{A Testsubstanz} - \text{A Testsubstanz ohne Zellen}}{\text{A Kontrolle} - \text{A Kontrolle ohne Zellen}} \times 100 \right)$$

mit

A Testsubstanz = Absorption der Wells mit Testsubstanz und mit Zellen
A Testsubstanz ohne Zellen = Absorption der Wells mit Testsubstanz ohne Zellen (Absorptionskontrolle)
A Kontrolle = Absorption der Wells ohne Testsubstanz, aber mit Zellen
A Kontrolle ohne Zellen = Absorption der Wells ohne Testsubstanz und ohne Zellen

Aus der Lipolyse-Inhibition [%] in einer Reihe von Verdünnungen von getesteten Proben wird der $IC_{50}$ berechnet. Dies ist die Konzentration, bei der die Lipolyse zu 50 % inhibiert wird.

Tabelle 5.2: Inhibition der Lipolyse durch die Einzelsubstanzen (Mittelwerte aus mindestens 2 unabhängigen Versuchen)

| **Testsubstanz** | **$IC_{50}$ (ppm)** |
|---|---|
| Santal (isoliert aus dem Kernholzextrakt von Rotsandelholz (*Pterocarpus santalinus*)) | 1,92 ppm |
| Formononetin (Fa. Phytolab) | 53,7 ppm |

**Beispiel 6:**

Die Wirksamkeit erfindungsgemäß zu verwendender Zubereitungen wurde an 30 Frauen (kaukasischer Typ) erprobt. Jede der Probandinnen behandelte zwei Monate lang ein Bein mit einer wie nachfolgend angegebenen erfindungsgemäß zu verwendenden Zubereitung und das andere Bein mit einer pterocarpanfreien Kontrollzubereitung. Nach zwei Monaten beurteilte ein Prüferpanel aus 3 geschulten Prüfern die Verbesserung des Cellulite-Erscheinungsbildes anhand einer Skala von 1 (gerade noch wahrnehmbare Verbesserung) bis 5 (vollständige Beseitigung des Cellulite-Bildes). Im Mittel wurde eine Verbesserung um 2 erzielt.

| | **Inhaltsstoff** | **INCI-Bezeichnung** | **Gew.-%** |
|---|---|---|---|
| A | Dracorin GOC | Glyceryl Oleate Citrate, Caprylic/Capric Triglyceride | 2,0 |
| | Neutralöl | Caprylic/Capric Triglyceride | 4,0 |
| | Paraffinöl | Paraffinum Liquidum | 4,0 |
| | PCI-Liquid 100 | Cetearyl Ethylhexanoate | 7,0 |
| | Dragoxat 89 | Ethylhexyl Isononanoate | 3,0 |
| | Dow Corning 345 Fluid | Cyclomethicone | 0,5 |

(fortgesetzt)

| | Inhaltsstoff | INCI-Bezeichnung | Gew.-% |
|---|---|---|---|
| | Dragosantol 100 | Bisabolol | 0,1 |
| **B** | Wasser | Water (Aqua) | 73,7 |
| | Pemulen TR2 | Acrylates/C10-30 Alkyl Acrylate Crosspolymer | 0,2 |
| **C** | Hydrolite-5 | Pentylene Glycol | 5,0 |
| | 3-Methoxypterocarpan (Verbindung B) | | 0,1 |
| **D** | Natrium Hydroxid 10%ige Lösung | Sodium Hydroxide | 0,4 |

Herstellung:

Pemulen TR2 in Wasser quellen lassen und 3-Methoxypterocarpan in Hydrolite-5 vorlösen. Phase A mischen. Phase C zu Phase A geben, anschließend Phase B zu Phase A/C zufügen und mit dem Homorex emulgieren. Die O/W mit dem Blattrührer nachrühren und dabei Phase D zugeben.

**FORMULIERUNGSBEISPIELE:**

| Beispiel Nr. | Produktform |
|---|---|
| 1 | Duschbad |
| 2 | Anti-Cellulite Körperöl |
| 3 | Körper-Spray O/W |
| 4 | Creme O/W |
| 5 | Körperlotion O/W |
| 6 | Anti-Cellulite Gel |
| 7 | Körperpeeling |
| 8 | Anti-Cellulite Balm |
| 9 | Feuchtiqkeits-Stick |
| 10 | Anti-Cellulite Spray-Gel |

| Inhaltsstoffe | INCI-Name | Gew.% | | | | | | | | | |
|---|---|---|---|---|---|---|---|---|---|---|---|
| | | 1 | 2 | 3 | 4 | 5 | 6 | 7 | 8 | 9 | 10 |
| **3-Methoxypterocarpan (B)** | | 0,1 | 0,02 | 0,05 | 0,08 | 0,02 | 0,1 | 0,08 | 0,05 | 0,1 | 0,1 |
| **oder** | | | | | | | | | | | |
| **Pao Rosa-Kernholz Extrakt** | | 0,2 | 0,02 | 0,1 | 0,16 | 0,04 | 0,2 | 0,16 | 0,1 | 0,2 | 0,2 |
| | | | | | | | | | | | |
| A-C Polyethylen 9 A | Polyethylene | | | | | | | 5 | | | |
| Actipone Black Coffee GW | Water (Aqua), Glycerin, Coffea Arabica (Coffee) Seed Extract, Coffea Robusta Seed Extract | | | | 1 | | | | | | |
| Actipone Laminaria Saccharina | Glycerin, Water (Aqua), Laminaria Saccharina Extract | | | | | | | | | 0,5 | |
| Actipone Nutgrass (Motha) Root GW | Water (Aqua), Glycerin, Cyperus Rotundus Root Extract | | | | | | 1 | | | | |
| Aristoflex AVC | Ammonium Acryloyldimethyltaurate/ VP Copolymer | | | | | | 0,7 | | | | |
| Avocado Öl | Persea Gratissima (Avocado) Oil | | 2 | | 6 | | | | | | |
| Biotive Esculin Sesquihydrate | Esculin | | | | | | | | | | 0,3 |
| (-)-alpha-Bisabolol natürlich | Bisabolol | | | | | | | | 0,1 | | |
| Butylenglykol-1,3 | Butylene Glycol | | | | | | | | | 14,34 | 5 |
| Carbopol Ultrez-10 | Carbomer | | | | 0,3 | | | | | | |
| Carbopol Ultrez 21 | Acrylates/C10-30 Alkyl Acrylate Crosspolymer | | | | | 0,2 | | | | | |
| Carnitin | Carnitine | | | | | | | | 0,8 | | 0,5 |
| Cetiol SB 45 | Butyrospermum Parkii (Shea Butter) | | | | 2 | | | | | | |
| Citronensäure Lsg. 10% | Citric Acid | 0,3 | | | | | | 0,05 | 0,2 | | |
| Comperlan 100 | Cocamide MEA | 0,5 | | | | | | | | | |

(fortgesetzt)

| Inhaltsstoffe | INCI-Name | Gew.% | | | | | | | | | |
|---|---|---|---|---|---|---|---|---|---|---|---|
| | | 1 | 2 | 3 | 4 | 5 | 6 | 7 | 8 | 9 | 10 |
| Covi-Ox T-70 | Tocopherol | | 0,2 | | 0,1 | 0,1 | | | | | |
| Cutina GMS-V | Glyceryl Stearate | | | | 1 | | | | | | |
| D-Panthenol | Panthenol | | | | | | | 0,5 | | | |
| Dow Corning 200 (100cs) Silicone Fluid | Dimethicone | | | | | | | | 3 | | |
| Dow Corning 246 Fluid | Cyclohexasiloxane | | | | | 2 | | | | | |
| Dow Corning 345 Fluid | Cyclomethicone | | | 0,5 | | | | | | | |
| Dracorin 100 S.E.P. | Glyceryl Stearate, PEG-100 Stearate | | | | | | | 7 | | | |
| Dracorin CE | Glyceryl Stearate Citrate | | | | 3 | | | | | | |
| Dracorin GOC | Glyceryl Oleate Citrate, Caprylic/Capric Triglyceride | | | 2 | | | | | | | |
| Drago-Oat-Active | Water (Aqua), Butylene Glycol, Avena Sativa (Oat) Kernel Extract | | | 1 | | | | | | | |
| Dragocid Liquid | Phenoxyethanol, Methylparaben, Ethylparaben, Butylparaben, Propylparaben, Isobutylparaben | | | | 0,8 | 0,8 | | | 0,4 | | 0,8 |
| Dragoderm | Glycerin, Triticum Vulgare (Wheat) Gluten, Water (Aqua) | 1 | | | | | | | | | |
| Dragosantol 100 | Bisabolol | | | 0,1 | | 0,1 | 0,5 | | | | |
| Dragosine | Carnosine | | | | | | 0,05 | | | | |
| Dragoxat 89 | Ethylhexyl Isononanoate | | 10 | 3 | | 4 | 5 | | | | |
| Edeta B Powder | Tetrasodium EDTA | | | | | | | 0,1 | | | |
| Edeta BD | Disodium EDTA | 0,1 | | | | | | | | | |
| Emulgin B2 | Ceteareth-20 | | | | | | | 2 | | | |
| Emulsiphos | Potassium Cetyl Phosphate, Hydrogenated Palm Glycerides | | | | | 1,2 | | | | 1 | |

(fortgesetzt)

| Inhaltsstoffe | INCI-Name | Gew.% | | | | | | | | | |
|---|---|---|---|---|---|---|---|---|---|---|---|
| | | 1 | 2 | 3 | 4 | 5 | 6 | 7 | 8 | 9 | 10 |
| Ethanol 96%ig | Alcohol Denat. | | | | | | | | | | 10 |
| Etherisches Öl | Essential Oil | | | | | | 1 | | | | |
| Extrapone Butcher's broom GW P | Glycerin, Water (Aqua), Pentylene Glycol, Ruscus Aculeatus Root Extract | | | | | | | 1 | | | |
| Extrapone Ginkgo Biloba | Propylene Glycol, Water (Aqua), Ginkgo Biloba Leaf Extract, Glucose, Lactic Acid | | | 1 | | | | | | | |
| Extrapone Green Tea (Organic) GW | Water (Aqua), Glycerin, Camellia Sinensis Leaf Extract | | | | | | | 2 | | | |
| Extrapone Guarana | Water (Aqua), Propylene Glycol, Paullinia Cupana Seed Extract, Alcohol | 1 | | | | | | | | | |
| Extrapone Horse Chestnut | Propylene Glycol, Water (Aqua), Aesculus Hippocastanum (Horse Chestnut) Seed Extract, Glucose, Lactic Acid | | | | | | | | | | 1 |
| Extrapone Ivy | Propylene Glycol, Water (Aqua), Hedera Helix (Ivy) Leaf/ Stem Extract, Glucose, Lactic Acid | | | | | | 1 | | | | |
| Extrapone Orange Flower | Water (Aqua), Propylene Glycol, Citrus Aurantium Amara (Bitter Orange) Flower Extract | | | | | | 3 | | | 1 | |
| Extrapone Orange Peel | Water (Aqua), Propylene Glycol, Alcohol, Citrus Aurantium Dulcis (Orange) Peel Extract | | | | | | | 1 | | | |
| Extrapone Seaweed | Water (Aqua), Butylene Glycol, Fucus Vesiculosus Extract | | | | | | | | 2,5 | | |
| Frescolat MGA | Menthone Glycerin Acetal | | | 0,5 | | | | | | | |
| Frescolat ML | Menthyl Lactate | | | | | | | | | 0,5 | |
| Genapol LRO Liquid | Sodium Laureth Sulfate | 37 | | | | | | | | | |
| Glycerin | Glycerin | | | | 3 | 3 | | | | 4 | |
| Hydrolite 5 | Pentylene Glycol | | | 5 | | | 5 | 5 | | | 3 |

(fortgesetzt)

| Inhaltsstoffe | INCI-Name | Gew.% | | | | | | | | | |
|---|---|---|---|---|---|---|---|---|---|---|---|
| | | 1 | 2 | 3 | 4 | 5 | 6 | 7 | 8 | 9 | 10 |
| Hydroviton | Water (Aqua), Glycerin, Sodium Lactate, Lactic Acid, TEA-Lactate, Serine, Urea, Sorbitol, Sodium Chloride, Allantoin | | | 1 | | | | | | | |
| Isodragol | Triisononanoin | | 13 | | 4 | | | | | | |
| Isopropylpalmitat | Isopropyl-Palmitate | | | | | | | 3 | | | |
| Jojoba Oil Ethoxilate (Oxypon 328) | Peg-26 Jojoba Acid, Peg-26 Jojoba Alcohol | | | | | | | | 1 | | |
| Kakaobutter, pulverisiert | Theobroma Cacao (Cocoa) Seed Butter | | | | | 0,7 | | | | | |
| Kaliumsorbat | Potassium Sorbate | | | | 0,2 | | | | | | |
| Karion F | Sorbitol | | | | | | | 1 | | | |
| Keltrol CG-RD | Xanthan Gum | | | | 0,1 | 0,2 | | | | | |
| Koffein reinst | Caffeine | | | | | | | | 0,5 | | 0,2 |
| Lanette 16 | Cetyl Alcohol | | | | 2,5 | | | 3 | | | |
| Lanette O | Cetearyl Alcohol | | | | | 1,5 | | | | | |
| Lösungsvermittler | PEG-40 Hydrogenated Castor Oil, Trideceth-9, Water (Aqua) | | | | | | | | | | 0,5 |
| Makadamia-Nuss-Öl | Macadamia Ternifoia Seed Oil | | 0,5 | | 3 | | | | | | |
| Meersalz (Totes Meer) | Sea Salt (Maris Sal) | | | | | | | 1,5 | | | |
| Merquat 550 | Polyquaternium-7 | 0,5 | | | | | | | | | |
| Natriumbenzoat | Sodium Benzoate | 0,5 | | | | | | | | | |
| Natriumchlorid | Sodium Chloride | 1 | | | | | | | | | |
| Natriumhydroxid Lsg. 10% | Sodium Hydroxide | | | 0,4 | 0,45 | 0,6 | 0,01 | | | | 0,4 |

(fortgesetzt)

| Inhaltsstoffe | INCI-Name | Gew.% | | | | | | | | | |
|---|---|---|---|---|---|---|---|---|---|---|---|
| | | 1 | 2 | 3 | 4 | 5 | 6 | 7 | 8 | 9 | 10 |
| Natriumstearat | Sodium Stearate | | | | | | | | | 9 | |
| Neo Actipone White Tea | Camellia Sinensis Leaf Extract | | | 3 | | 1 | | | | | |
| Neo Heliopan BB | Benzophenone-3 | | | | 0,1 | | 0,1 | | | | |
| Neutralöl | Caprylic/Capric Triglyceride | | | 4 | | | | 10 | | | |
| Orangenblüten Extrakt | Citrus Aurantium Amara (Bitter Orange) Flower Extract | 1 | | 1 | | | | | | | |
| Oxynex K Liquid | PEG-8, Tocopherol, Ascorbyl Palmitate, Ascorbic Acid, Citric Acid | | | | | | | 0,1 | | | |
| Paraffinöl | Paraffinum Liquidum | | 49,6 3 | 4 | | | | | | | |
| Parfümöl | Parfum (Fragrance) | 0,5 | 0,5 | 0,2 | 0,3 | 0,3 | | 0,4 | 0,4 | | 0,2 |
| PCL-Liquid 100 | Cetearyl Ethylhexanoate | | 21 | 7 | 3 | 3 | 3 | | | | |
| PCL-Solid | Stearyl Heptanoate, Stearyl Caprylate | | | | 1,5 | | 1,5 | | | | |
| Pemulen TR-2 | Acrylates/C10-30 Alkyl Acrylate Crosspolymer | | | 0,2 | | | | | | | |
| Phytoconcentrole Shea Butter | Glycine Soja (Soybean) Oil, Butyrospermum Parkii (Shea Butter) | | 0,5 | | | | | | | | |
| Propylenglycol-1,2 | Propylene Glycol | | | | | | | | 2 | | |
| RonaCare Nicotinamide | Niacinamide | | | | 0,1 | | | | | | |
| Sepigel 305 | Polyacrylamide, C 13-14 Isoparaffin, Laureth-7 | | | | | | | | 2 | | |
| Stabileze QM | PVM / Ma Decadiene Crosspolymer | | | | | | | | | | 0,25 |
| SymCalmin | Butylene Glycol, Pentylene Glycol, Hydroxyphenyl Propamidobenzoic Acid | | | | | 1 | | | | | |
| Symdiol 68 | 1.2-Hexanediol, Caprylyl Glycol | | 1 | | | | | | | | |
| SymGlucan | Water (Aqua), Glycerin, Beta-Glucan | | | | | 1 | | | | | |

(fortgesetzt)

| Inhaltsstoffe | INCI-Name | Gew.% | | | | | | | | | |
|---|---|---|---|---|---|---|---|---|---|---|---|
| | | 1 | 2 | 3 | 4 | 5 | 6 | 7 | 8 | 9 | 10 |
| SymMatrix | Maltodextrin, Rubus Fruticosus (Blackberry) Leaf Extract | | | | | 0,5 | | | | | |
| SymMollient W/S | Trideceth-9, PEG-5 Isononanoate, Water (Aqua) | 1 | | | | | | | | | |
| SymPeptide 222 | Glycerin, Water (Aqua), Myristoyl Pentapeptide-8 | | | | | | | | 5 | | |
| SymRelief | Bisabolol, Zingiber Officinale (Ginger) Root Extract | | 0,1 | | | | | | | | |
| SymRepair | Hexyldecanol, Bisabolol, Cetylhydroxyproline Palmitamide, Stearic Acid, Brassica Campestris (Rapeseed) Sterols | | 1 | | | | | | | | |
| SymVital | Aloe Barbadensis Leaf Juice Powder, Magnesium Ascorbyl Phosphate, Rubus Idaeus (Raspberry) Leaf Extract | | | | | | | | 0,5 | | |
| Talkum | Talc | | | | | | | | 3 | | |
| Tego Betain L7 non preserved | Cocamidopropyl Betain | 6 | | | | | | | | | |
| Vitamin A Palmitat | Retinyl Palmitate | | 0,05 | | | | | | | | |
| Vitamin E Acetat | Tocopheryl Acetate | | 0,5 | | | 0,5 | | | | | |
| Wasser | Water (Aqua) | ad 100 | | ad 100 | ad 100 | ad 100 | ad 100 | ad 100 | ad 100 | ad 100 | ad 100 |

**Beispiele: F1 - F10: Oral konsumierbare Anwendungsbeispiele ["Schönheit von Innen" ("Beauty from Inside")]**

Beispiel F1: Fruchtgummis

| | Gew.-% |
|---|---|
| Wasser | Ad 100 |
| Saccharose | 34,50 |
| Glucosesirup, DE 40 | 31,89 |
| Iso Syrup C* Tru Sweet 01750 (Cerestar GmbH) | 1,50 |
| Gelatine 240 Bloom | 8,20 |
| Gelbe und rote Lebensmittelfarbstoffe | 0,01 |
| Citronensäure | 0,20 |
| 3-Methoxypterocarpan (B) | 0,075 |

Beispiel F2: Hardkaramelle (hard boiled candy)

| | I (Gew.-%) | II (Gew.-%) |
|---|---|---|
| Zucker (Saccharose) | Ad 100 | Ad 100 |
| Glucose-Fructose-Sirup (high fructose corn syrup) | 41,00 | 41,00 |
| Maltose | 3,00 | 3,00 |
| Palmkernöl | 0,90 | 0,90 |
| Citronensäure | 0,30 | 0,30 |
| Ingwerextrakt | 0,40 | - |
| Ginsengextrakt | - | 0,40 |
| Blauer Farbstoff | 0,01 | 0,01 |
| 3-Methoxypterocarpan (B) | 0,10 | - |
| Pao Rosa-Kernholz Extrakt | - | 0,16 |
| Honig | - | 1,50 |
| Honigaroma | - | 0,30 |

Beispiel F3: Zum Direktverzehr geeignete Gelatinekapseln

| | Gew.-% | | |
|---|---|---|---|
| | I | II | III |
| Gelatinehülle: | | | |
| Glycerin | 2,014 | 2,014 | 2,014 |
| Gelatine 240 Bloom | 7,91 | 7,91 | 7,91 |
| Aspartam | 0,05 | - | - |

(fortgesetzt)

| | Gew.-% | | |
|---|---|---|---|
| | I | II | III |
| Sucralose | 0,035 | 0,050 | 0,070 |
| Allura Red (roter Farbstoff) | 0,006 | 0,006 | 0,006 |
| Brillant Blue (blauer Farbstoff) | 0,005 | 0,005 | 0,005 |
| | | | |
| Kernzusammensetzung: | | | |
| Pflanzenöltriglyceride (Kokonussölfraktion; coconut oil fraction) | Ad 100 | Ad 100 | Ad 100 |
| Aroma G | 9,95 | 12,0 | 12,0 |
| 3-Methoxypterocarpan (Verbindung (B)) | 0,07 | 0,10 | - |
| Pao Rosa-Kernholz Extrakt | - | - | 0,20 |

Aroma G hatte dabei folgende Zusammensetzung (Angaben jeweils in Gew.-%): 0,1% Neotam Pulver, 29,3% Pfefferminzöl arvensis, 29,35% Pfefferminz piperita Öl Willamette, 2,97% Sucralose, 2,28% Triacetin, 5,4% Diethyltartrat, 12,1% Pfefferminzöl yakima, 0,7% Ethanol, 3,36% 2-Hydroxyethylmenthylcarbonat, 3,0% 2-Hydroxypropylmenthylcarbonat, 5,77% D-Limonen, 5,67% L-Menthylacetat.

Die zum Direktverzehr geeigneten Gelatinekapseln I, II, III wurden gemäß WO 2004/050069 hergestellt und hatten jeweils einen Durchmesser von 5 mm, das Gewichtsverhältnis von Kernmaterial zu Hüllmaterial lag bei 90 : 10. Die Kapseln öffneten sich jeweils im Mund innerhalb von weniger als 10 Sekunden und lösten sich vollständig innerhalb von weniger als 50 Sekunden auf.

Beispiel F4: Komprimate in runder Tablettenform

| | Gew.-% | | |
|---|---|---|---|
| | I | II | III |
| Magnesiumstearat | 0,9 | 0,9 | 0,9 |
| Citronensäure | 0,2 | 0,2 | 0,2 |
| 3-Methoxypterocarpan (B) | 0,05 | 0,10 | - |
| Pao Rosa-Kernholz Extrakt | - | - | 0,23 |
| Dextrose | Ad 100 | Ad 100 | Ad 100 |

Beispiel F5: Kaugummis (mit Zucker und zuckerfrei)

| | Gew.-% | |
|---|---|---|
| | I | II |
| Kaugummibase | 21,0 | 30,0 |
| Glycerin | 0,5 | 1,0 |
| Menthol-Spearmint-Eucalyptus Aroma P1 | 1,0 | 1,4 |
| Glucosesirup | 16,5 | - |
| Puderzucker | Ad 100 | - |

(fortgesetzt)

| | Gew.-% | |
|---|---|---|
| | I | II |
| 3-Methoxypterocarpan (Verbindung (B)) | 0,15 | 0,20 |
| Sorbitol (in Pulverform) | | Ad 100 |
| Palatinit | | 9,5 |
| Xylitol | | 2,0 |
| Mannitol | | 3,0 |
| Aspartam | | 0,1 |
| Acesulfam K | | 0,1 |
| Emulgum (Emulgator) | | 0,3 |
| Sorbitol 70%, in Wasser | | 14,0 |

Aroma P1 hatte folgende Zusammensetzung (Angaben jeweils in Gew.-%):
0,05% Isobutyraldehyd, 0,05% 3-Octanol, 0,05% Dimethylsulfid, 0,1% trans-2-Hexenal, 0,1% cis-3-Hexenol, 0,1% natürliches 4-Terpineol, 0,1% Isopulegol, 0,2% natürliches Piperiton, 0,3% Linalool, 1,0% Isoamylalkohol, 1,0% Isovaleraldehyd, 2,5% natürliches alpha-Pinen, 2,5% natürliches beta-Pinen, 8,0% Eucalyptol, 7,0% I-Menthylacetat, 12,0% I-Menthon, 5,0% Isomenthon, 20,5% I-Carvon, 39,45% I-Menthol.

Die nachfolgende Tabelle betrifft Beispiele F6 -F10:

Beispiel F6 = Instant Getränkepulver

Beispiel F7 = Instant Getränkepulver, zuckerfrei

Beispiel F8 = Carbonisierte Limonade (soft drink)

Beispiel F9 = Soja-Fruchtgetränk

Beispiel F10 = Fettreduzierter Joghurt

| | Gew.-% | | | | |
|---|---|---|---|---|---|
| | F6 | F7 | F8* | F9 | F10 |
| 3-Methoxypterocarpan (B) | 0,50 | 0,90 | 0,05 | 0,05 | 0,10 |
| Pao Rosa-Kernholz Extrakt | - | - | - | 0,08 | - |
| Zucker (Saccharose) | Ad 100 | | | | |
| Citronensäure | 4,00 | 33,33 | 0,2 | | |
| Trinatriumcitrat | 0,26 | | | | |
| Tricalciumphosphat | 0,22 | | | | |
| Ascorbinsäure (Vitamin C) | 0,24 | 0,44 | | | |
| Trübungsmittel und Titandioxid (E 171) | 0,20 | | | | |
| Xanthan gum (E 415) | 0,072 | | | | |
| Natriumcarboxymethylcellul ose (E 467) | 0,064 | | | | |

(fortgesetzt)

| | Gew.-% | | | | |
|---|---|---|---|---|---|
| | F6 | F7 | F8* | F9 | F10 |
| Pectin (E 440) | 0,04 | | | | |
| Sprühgetrocknetes Ananas-Aroma, enthält gelben Farbstoff Tartrazin | 0,40 | | | | |
| Sprühgetrocknetes Himbeer-Aroma, enthält roten Farbstoff | | 11,50 | | | |
| Citronen-Limetten-Aroma | | | 0,01 | | |
| D-Limonen | | | 0,005 | | |
| Maltodextrin (Pulver) | | Ad 100 | | | |
| Aspartam | | 3,30 | | | |
| Saccharose | | | 8,0 | 6,0 | 5,0 |
| Hesperetin (1 Gew.-% in 1,2-Propyleneglykol) | | | 0,05 | | |
| Ethylhydroxymethylfuranon | | | 0,01 ppb | | |
| Vanillearoma | | | | 0,10 | 0,125 |
| Vanillin | | | 15 ppb | | |
| Maltol | | | 350 ppb | | |
| 2,5-Dimethyl-4-hydroxy-2H-furan-3-on | | | 3 ppb | | |
| 1,2-Propyleneglykol | | | 0,1 | | |
| Mischung von Fruchtsaftkonzentraten | | | | 45,0 | |
| Sojapulver | | | | 5,0 | |
| Joghurt (1,5 Gew.-% Fett) | | | | | Ad 100 |
| Wasser | | | Ad 100 | Ad 100 | |
| * Versetzen mit Kohlensäure nach Abfüllung in Flaschen. | | | | | |

**Patentansprüche**

1. Nicht-therapeutische, kosmetische Verwendung einer Zubereitung, enthaltend eine, zwei oder mehr Verbindungen der Formel (I) und/oder ein pharmazeutisch akzeptables Salz einer solchen Verbindung,

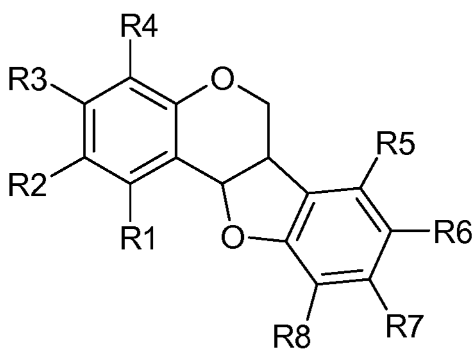

(I)

zur Vorbeugung, Behandlung oder Verringerung von Cellulite,

wobei die Reste R1 bis R8, unabhängig voneinander, Wasserstoff, Hydroxy oder C1-C4-Alkoxy bedeuten, und/oder zwei benachbarte Reste gemeinsam eine Methylendioxygruppe bilden.

2. Verwendung einer Zubereitung nach Anspruch 1, wobei die Reste R1 bis R8, unabhängig voneinander, Wasserstoff, Hydroxy, Methoxy oder Ethoxy bedeuten und optional ein oder mehrere Paare benachbarter Reste jeweils gemeinsam eine Methylendioxygruppe bilden.

3. Verwendung einer Zubereitung nach einem der vorherigen Ansprüche, wobei eine Verbindung der Formel (I) eine Verbindung der Formel (IA) ist,

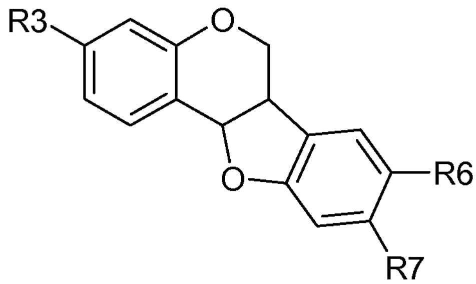

(IA)

wobei der Rest R3 Wasserstoff, Hydroxy oder Methoxy bedeutet, und die Reste R6 und R7 gemeinsam eine Methylendioxygruppe bilden oder unabhängig voneinander Wasserstoff, Hydroxy oder Methoxy bedeuten.

4. Verwendung einer Zubereitung nach einem der vorherigen Ansprüche, wobei eine, mehrere oder alle Verbindungen der Formel (I) ausgewählt sind aus der Gruppe bestehend aus Medicarpin, Maackiain, Pterocarpin, Homopterocarpin und 3-Methoxypterocarpan.

5. Verwendung einer Zubereitung nach einem der vorherigen Ansprüche, mit einem Photo-Irritationsfaktor gemäß OECD Guideline 432 in der am 13. April 2004 angenommenen Fassung von weniger als 5, und/oder mit einem Gehalt an methoxylierten Isoflavonen von nicht mehr als 16 Gew.-% bezogen auf die Gesamt-Trockenmasse.

6. Verwendung einer Zubereitung nach einem der vorherigen Ansprüche, wobei die Zubereitung ferner ein oder mehrere Lipolyse-Stimulatien umfasst, wobei einer, mehrere oder alle der Lipolyse-Stimulantien

- eine Verbindung der Formel (Xa) oder ein pharmazeutisch akzeptables Salz einer solchen Verbindung ist,

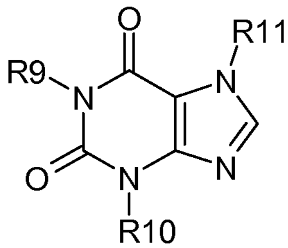

(Xa)

wobei R9, R10 und R11 unabhängig voneinander Wasserstoff oder Methyl bedeuten, und/oder
- eine Verbindung der Formel (PhEA) oder ein pharmazeutisch akzeptables Salz einer solchen Verbindung ist,

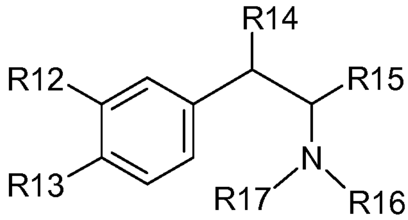

(PhEA)

wobei, unabhängig voneinander,

R12 und R13 jeweils Wasserstoff, Hydroxy und Methoxy,
R14 Wasserstoff, Hydroxy und Methyl,
R15 Wasserstoff und Methyl und
R16 und R17 jeweils Wasserstoff und C1-C4-Alkyl bedeuten.

**7.** Verwendung einer Zubereitung nach Anspruch 6, wobei eine mehrere oder alle Verbindungen der Formel (PhEA) Verbindungen der Formel (PhEA-i) sind, und deren pharmazeutisch akzeptable Salze,

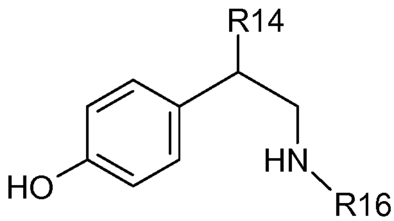

(PhEA-i)

wobei der Rest R14 Wasserstoff, Hydroxy und Methyl und der Rest R16 Wasserstoff und C1-C4-Alkyl bedeuten.

**8.** Verwendung einer Zubereitung nach einem der vorherigen Ansprüche, wobei

- die Gesamtmenge der Verbindungen der Formel (I) in der Zubereitung 0,001 - 1 Gew.-%, vorzugsweise 0,005 - 0,5 Gew.-%, besonders bevorzugt 0,01 - 0,2 Gew.-% und insbesondere bevorzugt 0,02 - 0,1 Gew.-% beträgt, und/oder
- die Gesamtmenge der Verbindungen der Formel (Xa) in der Zubereitung 0,005 - 10 Gew.-%, vorzugsweise 0,05 - 5 Gew.-% und besonders bevorzugt 0,5 - 2,5 Gew.-% beträgt, und/oder
- die Gesamtmenge an Agonisten beta-adrenerger Rezeptoren in der Zubereitung 0,0001 - 0,1 Gew.-%, vorzugsweise 0,001 - 0,05 Gew.-% und besonders bevorzugt 0,002
- 0,02 Gew.-% beträgt,

jeweils bezogen auf die gesamte Zubereitung.

**9.** Verwendung einer Zubereitung nach einem der Ansprüche 6 bis 8, wobei
das Gewichtsverhältnis der Gesamtmenge der Verbindungen der Formel (I) zur Gesamtmenge der Verbindungen der Formel (Xa) in der Zubereitung 1:1 bis 1:500, vorzugsweise 1:5 bis 1:125 beträgt, und/oder
das Gewichtsverhältnis der Gesamtmenge der Verbindungen der Formel (I) zur Gesamtmenge der Agonisten beta-adrenerger Rezeptoren in der Zubereitung 100:1 bis 1:5, vorzugsweise 50:1 bis 1:1 und besonders bevorzugt 25:1 bis 2:1 beträgt.

**10.** Kosmetische, dermatologische oder pharmazeutische Zusammensetzung enthaltend

(a) einen Pao Rosa-Kernholz-Extrakt, der mit Aktivkohle behandelt wurde, enthaltend eine, zwei oder mehr Verbindung(en) der Formel (I) und/oder ein pharmazeutisch akzeptables Salz einer solchen Verbindung,

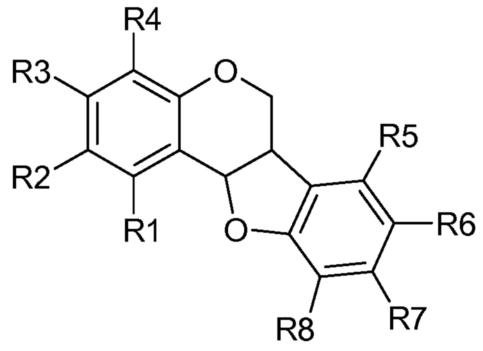

(I)

wobei die Reste R1 bis R8, unabhängig voneinander, Wasserstoff, Hydroxy oder C1-C4-Alkoxy bedeuten, und/oder zwei benachbarte Reste gemeinsam eine Methylendioxygruppe bilden,
wobei die Gesamtmenge der Verbindungen der Formel (I) 0,001 - 1 Gew.-% bezogen auf die Gesamtzusammensetzung beträgt
und umfassend
(b) 0,005 - 10 Gew.-% bezogen auf die Gesamtzusammensetzung eines oder mehrerer Lipolyse-Stimulans/Stimulatien der Formel (Xa)

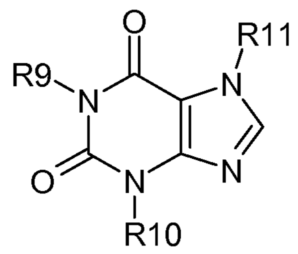

(Xa)

wobei R9, R10 und R11 unabhängig voneinander Wasserstoff oder Methyl bedeuten.

**11.** Nicht-therapeutische, kosmetische Verwendung einer Verbindung der Formel (I) und/oder eines pharmazeutisch akzeptablen Salzes einer solchen Verbindung,

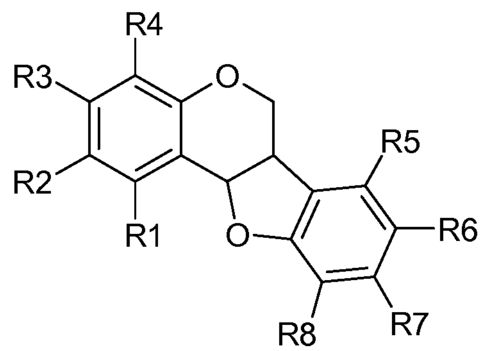

(I)

zur Vorbeugung, Behandlung oder Verringerung von Cellulite,
wobei die Reste R1 bis R8, unabhängig voneinander, Wasserstoff, Hydroxy oder C1-C4-Alkoxy bedeuten, und/oder zwei benachbarte Reste gemeinsam eine Methylendioxygruppe bilden.

## Claims

1. Non-therapeutic, cosmetic use of a preparation, containing one, two or more compounds of formula (I) and/or a pharmaceutically acceptable salt of such a compound,

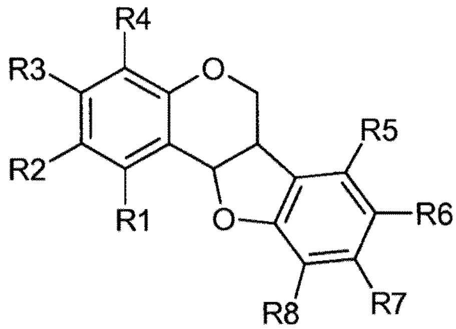

(I)

for the prevention, treatment or reduction of cellulite,
wherein the rests R1 to R8 denote, independent of each other, hydrogen, hydroxyl or C1-C4-alkoxy and/or two neighbouring rests together form a methylenedioxygroup.

2. Use of a preparation according to claim 1, wherein the rests R1 to R8 denote, independent of each other, hydrogen, hydroxyl, methoxy or ethoxy and optionally one or more pairs of neighbouring rests each together form a methylenedioxygroup.

3. Use of a preparation according to any of the previous claims, wherein a compound of formula (I) is a compound of formula (IA),

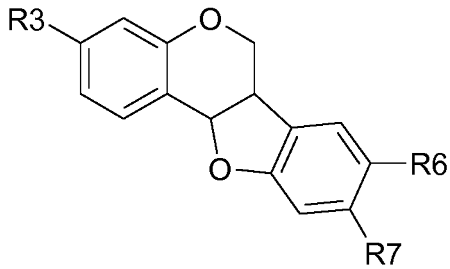

(IA)

wherein the rest R3 denotes hydrogen, hydroxyl or methoxy and the rests R6 and R7 together form a methylenedioxygroup or denote independent of each other hydrogen, hydroxyl or methoxy.

4. Use of a preparation according to any of the previous claims, wherein one, more or all compounds of formula (I) are selected from the group consisting of medicarpin, maackiain, pterocarpin, homopterocarpin and 3-methoxypterocarpan.

5. Use of a preparation according to any of the previous claims with a photo-irritation factor according to OECD guideline 432 in the version adopted on 13 April 2004 of less than 5 and/or with a content of methoxylated isoflavones of not more than 16 wt.-% with regard to the total dry mass.

6. Use of a preparation according to any of the previous claims, wherein the preparation further comprises one or more lipolysis stimulants, wherein one, more or all of the lipolysis stimulants

- is a compound of formula (Xa) or a pharmaceutically acceptable salt of such a compound,

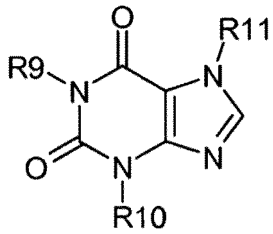

(Xa)

wherein R9, R10 and R11 denote independent of each other hydrogen or methyl, and/or
- is a compound of formula (PhEA) or a pharmaceutically acceptable salt of such a compound,

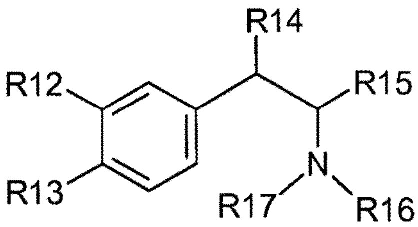

(PhEA)

Wherein, independent of each other,

R12 and R13 each denote hydrogen, hydroxyl and methoxy,
R4 denotes hydrogen, hydroxyl and methyl,
R15 denotes hydrogen and methyl and
R16 and R17 each denote hydrogen and C1-C4-Alkyl.

**7.** Use of a preparation according to claim 6, wherein one, more or all compounds of formula (PhEA) are compounds of formula (PhEA-i) and their pharmaceutically acceptable salts,

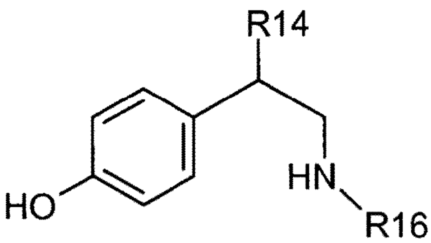

(PhEA-i)

wherein the rest R14 denotes hydrogen, hydroxyl and methyl and the rest R16 denotes hydrogen and C1-C4-alkyl.

**8.** Use of a preparation according to any of the previous claims, wherein

- the total amount of compounds of formula (I) in the preparation is 0.001 - 1 wt.-%, preferably 0.005 - 0.5 wt.-%, particularly preferably 0.01 - 0.2 wt.-% and especially preferably 0.02 - 0.1 wt.-%, and/or

- the total amount of compounds of formula (Xa) in the preparation is 0.005 - 10 wt.-%, preferably 0.05 - 5 wt.-% and particularly preferably 0.5 - 2.5 wt.-%, and/or
- the total amount of agonists of beta-adrenergic receptors in the preparation is 0.0001 - 0.1 wt.-%, preferably 0.001 - 0.05 wt.-% and particularly preferably 0.002 - 0.02 wt.-%,

each with regard to the total preparation.

**9.** Use of a preparation according to one of the claims 6 to 8, wherein
the weight ratio of the total amount of compounds of formula (I) to the total amount of compounds of formula (Xa) in the preparation is 1:1 to 1:500, preferably 1:5 to 1:125, and/or
the weight ratio of the total amount of compounds of formula (I) to the total amount of agonists of beta-adrenergic receptors in the preparation is 100:1 to 1:5, preferably 50:1 to 1:1 and particularly preferably 25:1 to 2:1.

**10.** Cosmetic, dermatological or pharmaceutical composition comprising

(a) a Pao Rose-heartwood-extract, which was treated with activated carbon, comprising one, two or more compound(s) of formula (I) and/or a pharmaceutical acceptable salt of such a compound

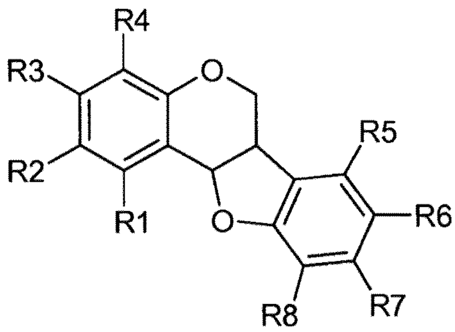

(I)

wherein the rests R1 to R8 denote, independent of each other, hydrogen, hydroxyl or C1-C4-alkoxy, and/or two neighbouring rests together form a methylenedioxygroup,
wherein the total amount of compounds of the formula (I) is 0.001 - 1 wt.-% with regard to the total composition and comprising
(b) 0.005 - 10 wt.-% with regard to the total composition of one or more lipolysis stimulant/stimulants of formula (Xa)

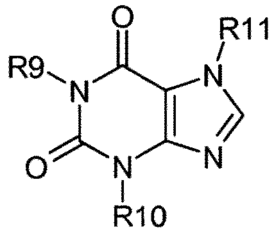

(Xa)

wherein R9, R10 and R11 denote independent of each other hydrogen or methyl.

**11.** Non-therapeutic, cosmetic use of a compound of formula (I) and/or of a pharmaceutically acceptable salt of such a compound,

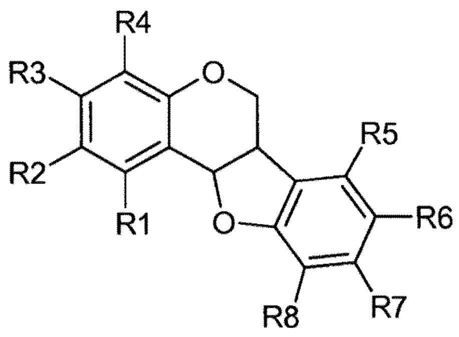

(I)

for the prevention, treatment or reduction of cellulite,
wherein the rests R1 to R8 denote, independent of each other, hydrogen, hydroxyl or C1-C4-alkoxy, and/or two neighbouring rests together form a methylenedioxy-group.

## Revendications

**1.** Utilisation cosmétique non-thérapeutique d'une préparation contenant un, deux ou plusieurs composés de formule (I) et/ou un sel pharmaceutiquement acceptable d'un tel composé

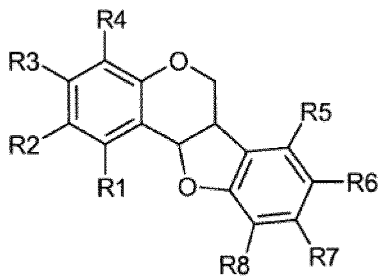

(I)

pour la prévention, le traitement ou la réduction de la cellulite,
dans laquelle les radicaux R1 à R8 représentent, indépendamment les uns des autres, de l'hydrogène, un groupe hydroxy ou alcoxy en C1 à C4, et/ou deux radicaux adjacents forment ensemble un groupe méthylènedioxy.

**2.** Utilisation d'une préparation selon la revendication 1, dans laquelle les radicaux R1 à R8 représentent, indépendamment les uns des autres, de l'hydrogène, un groupe hydroxy, méthoxy ou éthoxy, et, optionnellement, une ou plusieurs paires de radicaux adjacents forment respectivement ensemble un groupe méthylènedioxy.

**3.** Utilisation d'une préparation selon l'une quelconque des revendications précédentes, dans laquelle un composé de formule (I) est un composé de formule (IA),

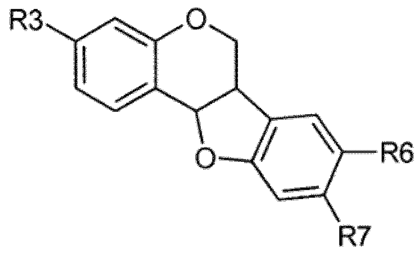

(IA)

dans laquelle le radical R3 représente de l'hydrogène, un groupe hydroxy ou méthoxy, et les radicaux R6 et R7 forment ensemble un groupe méthylènedioxy ou représentent, indépendamment l'un de l'autre, de l'hydrogène, un groupe hydroxy ou méthoxy.

**4.** Utilisation d'une préparation selon l'une quelconque des revendications précédentes, dans laquelle un, plusieurs ou l'ensemble des composés de formule (I) sont choisis dans le groupe constitué par la médicarpine, la maackiaïne, la ptérocarpine, l'homoptérocarpine et le 3-méthoxyptérocarpane.

**5.** Utilisation d'une préparation selon l'une quelconque des revendications précédentes, avec un facteur de photo-irritation inférieur à 5, selon la ligne directrice 432 de l'OCDE dans la version adoptée le 13 avril 2004, et/ou avec une teneur en isoflavones méthoxylées n'excédant pas 16 % en poids par rapport à la matière sèche totale.

**6.** Utilisation d'une préparation selon l'une quelconque des revendications précédentes, la préparation comprenant en outre un ou plusieurs stimulants de la lipolyse, un, plusieurs ou l'ensemble des stimulants de la lipolyse

- étant un composé de formule (Xa) ou un sel pharmaceutiquement acceptable d'un tel composé,

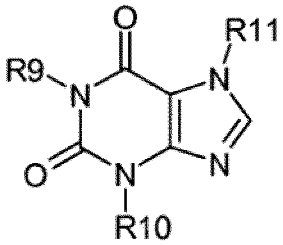

(Xa)

dans laquelle R9, R10 et R11 représentent, indépendamment les uns des autres, de l'hydrogène ou un groupe méthyle, et/ou
- un composé de formule (PhEA), ou un sel pharmaceutiquement acceptable d'un tel composé,

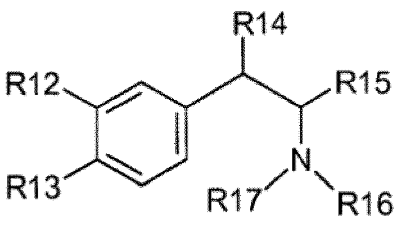

(PhEA)

dans laquelle, indépendamment les uns des autres,

R12 et R13 représentent respectivement de l'hydrogène, un groupe hydroxy et un groupe méthoxy,
R14 représente de l'hydrogène, un groupe hydroxy et un groupe méthyle,
R15 représente de l'hydrogène et un groupe méthyle et
R16 et R17 représentent respectivement de l'hydrogène et un groupe alkyle en C1 à C4.

**7.** Utilisation d'une préparation selon la revendication 6, dans lequel un, plusieurs ou l'ensemble des composés de formule (PhEA) sont des composés de formule (PhEA-i), et leurs sels pharmaceutiquement acceptables,

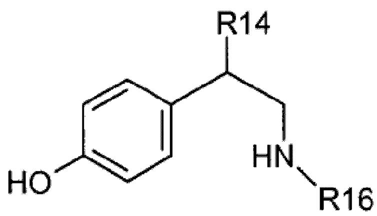

(PhEA-i)

dans laquelle le radical R14 représente de l'hydrogène, un groupe hydroxy et un groupe méthyle, et le radical R16 représente de l'hydrogène et un groupe alkyle en C1 à C4.

**8.** Utilisation d'une préparation selon l'une quelconque des revendications précédentes, dans laquelle

- la quantité totale des composés de formule (I) dans la préparation est comprise entre 0,001 et 1 % en poids, de préférence entre 0,005 et 0,5 % en poids, de manière particulièrement préférée entre 0,01 et 0,2 % en poids et en particulier de préférence entre 0,02 et 0,1 % en poids, et/ou
- la quantité totale des composés de formule (Xa) dans la préparation est comprise entre 0,005 et 10 % en poids, de préférence entre 0,05 et 5 % en poids et de manière particulièrement préférée entre 0,5 et 2,5 % en poids, et/ou
- la quantité totale d'agonistes de récepteurs bêta-adrénergiques dans la préparation est comprise entre 0,0001 et 0,1 % en poids, de préférence entre 0,001 et 0,05 % en poids, et de manière particulièrement préférée entre 0,002 et 0,02 % en poids,

respectivement par rapport à la préparation totale.

**9.** Utilisation d'une préparation selon l'une quelconque des revendications 6 à 8, dans laquelle
le rapport pondéral de la quantité totale des composés de formule (I) à la quantité totale des composés de formule (Xa) dans la préparation est compris entre 1 : 1 et 1 : 500, de préférence entre 1 : 5 et 1 : 125, et/ou
le rapport pondéral de la quantité totale des composés de formule (I) à la quantité totale des agonistes de récepteurs bêta-adrénergiques dans la préparation est compris entre 100 : 1 et 1 : 5, de préférence entre 50 : 1 et 1 : 1 et de manière particulièrement préférée entre 25 : 1 et 2 : 1.

**10.** Composition cosmétique, dermatologique ou pharmaceutique contenant

(a) un extrait de bois de coeur Pao Rosa qui a été traité avec du charbon actif, contenant un, deux ou plusieurs composé(s) de formule (I) et/ou un sel pharmaceutiquement acceptable d'un tel composé,

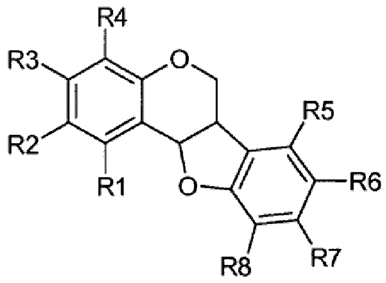

(I)

dans laquelle les radicaux R1 à R8 représentent, indépendamment les uns des autres, de l'hydrogène, un groupe hydroxy ou alcoxy en C1 à C4, et/ou deux radicaux adjacents forment ensemble un groupe méthylènedioxy,
la quantité totale des composés de formule (I) étant comprise entre 0,001 et 1 % en poids par rapport à la composition totale,
et comprenant
(b) de 0,005 à 10 % en poids, par rapport à la composition totale, d'un ou de plusieurs stimulant(s) de la lipolyse de formule (Xa)

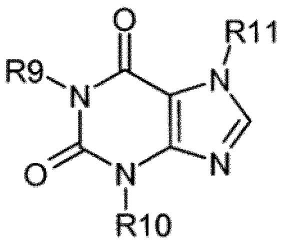

(Xa)

dans laquelle R9, R10 et R11 représentent, indépendamment les uns des autres, de l'hydrogène ou un groupe méthyle.

**11.** Utilisation cosmétique non-thérapeutique d'un composé de formule (I) et/ou d'un sel pharmaceutiquement acceptable d'un tel composé,

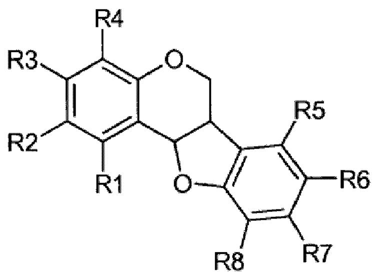

(I)

pour la prévention, le traitement ou la réduction de la cellulite,
dans laquelle les radicaux R1 à R8 représentent, indépendamment les uns des autres, de l'hydrogène, un groupe hydroxy ou alcoxy en C1 à C4, et/ou deux radicaux adjacents forment ensemble un groupe méthylènedioxy.

Figur 1

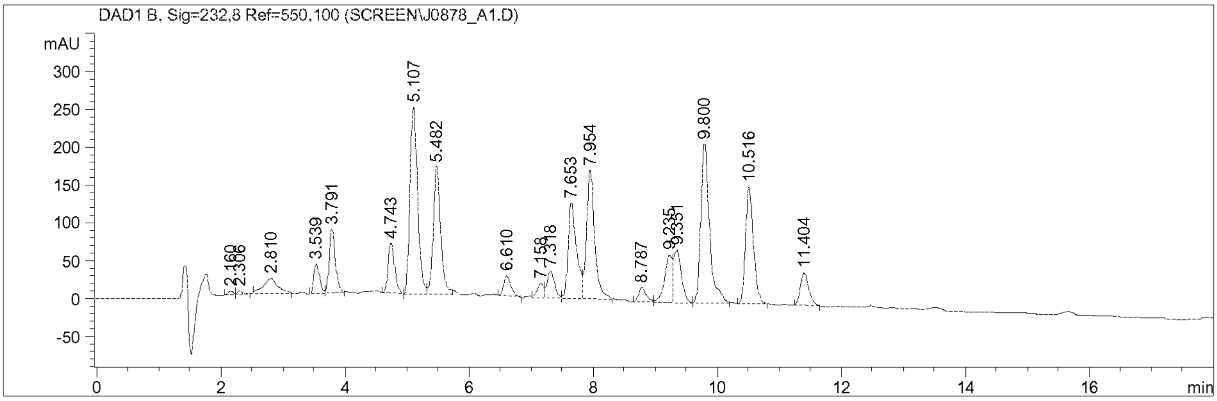


Figur 2

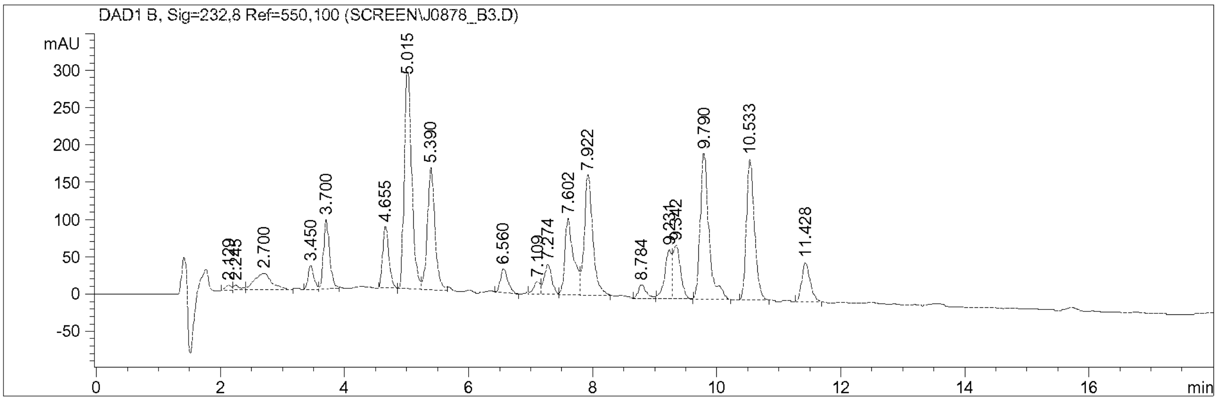

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1234572 A
- DE 102004032837
- DE 10009423
- WO 0197771 A2
- US 6030620 A
- US 5721371 A
- JP 2002053421 A
- US 4704400 F
- WO 2007128725 A
- US 5929124 A
- WO 2007128725 A1
- WO 2005123101 A1
- WO 02069992 A
- US 20060089413 A
- WO 2008046791 A
- WO 2005123101 A
- WO 2008046795 A
- WO 2007042472 A
- US 20080070825 A
- WO 0176572 A
- WO 0215686 A
- WO 2008046676 A
- WO 2006053912 A
- WO 2007110415 A
- WO 2005107692 A
- WO 2006015954 A
- WO 2007128723 A
- WO 2007060256 A
- WO 2006045760 A
- EP 0389700 A
- US 4251195 A
- US 6214376 B
- WO 03055587 A
- WO 2004050069 A
- US 4150052 A
- WO 2005049553 A
- WO 2004026840 A


### In der Beschreibung aufgeführte Nicht-Patentliteratur


- *Cellular Signalling,* 2006, vol. 18, 401-8
- *Laut Biochem. Pharmacol.,* 1992, vol. 44, 1307-1315
- *J. Liquid Chromatography & Related Technologies,* 2005, vol. 28, 823-834
- *Anlehnung an Tetrahedron,* 1999, vol. 55, 11773-11786
- *Anlehnung an Synlett,* 1994, 1001-1002
- Perkin Trans. *J. Chem. Soc.,* 1998, vol. 1, 2533-2536
- *J. Nat. Prod.,* 2008, vol. 71, 275-277
- **KIRKBRIDE et al.** *Brittonia,* 1997, 1-23
- Afrikanische Arzneipflanzen und Jagdgifte. **HANS DIETER NEUWINGER.** Chemie, Pharmakologie, Toxikologie. Wiss. Verl.-Ges, 1998, 316-321
- **SUTER et al.** *Acta Tropica,* 1986, 69-83
- **BOREL et al.** *Helvetica Chimica Acta,* 1987, 570-576
- **GEORGES et al.** *Bioresource Technology,* 2008, 2037-2045
- *Journal of Ethnopharmacology,* 2007, 99-104
- *Journal of Ethnopharmacology,* 2004, 43-49
- **MAGASSOUBA et al.** *Journal of Ethnopharmacology,* 2007, 44-53
- **HARPER et al.** *J. Chem. Soc.,* 1969, 1109-1116
- **S. ARCTANDER.** Perfume and Flavor Chemicals. Eigenverlag, 1969
- **SURBURG ; PANTEN.** Common Fragrance and Flavor Materials. Wiley-VCH, 2006